# EUROPEAN PATENT SPECIFICATION

(11) **EP 2 992 053 B1**
(45) Date of publication and mention of the grant of the patent: **28.11.2018**
(21) Application number: 14727409.6
(22) Date of filing: 29.04.2014
(51) Int. Cl.: C09B 29/08, C09B 1/54, C09B 1/26, C09B 57/00, C09B 1/516, A61Q 5/06, B27K 5/02

(54) **COLORING AGENTS NATURALISED WITH THE 6'-DEOXY-6'-(PIPERAZINYL)LACTOSE MOIETY**
MIT DEM 6'-DEOXY-6'-(PIPERAZINYL)LACTOSE-TEIL NATURALISIERTE FÄRBEMITTEL
COLORANTS RENDUS À L'ÉTAT NATUREL AU MOYEN DE LA FRACTION 6'-DÉSOXY-6'-(PIPÉRAZINYL)LACTOSE

(30) Priority: 29.04.2013 IT FI20130093
(43) Date of publication of application: 09.03.2016
(73) Proprietor: Universita' Degli Studi di Firenze, 50121 Firenze (IT)
(72) Inventor: BIANCHINI, Roberto, I-50019 Sesto Fiorentino (IT); CORSI, Massimo, I-50019 Sesto Fiorentino (IT); BONANNI, Marco, I-50019 Sesto Fiorentino (IT)
(74) Representative: Valenza, Silvia
(86) International application number: PCT/EP2014/058649
(87) International publication number: WO 2014/177528

(56) References cited:
- EP-A1- 1 462 484
- EP-A1- 2 085 434

## Description

### FIELD OF THE INVENTION

The present application refers to water soluble and multifunctional dyeing agents.

### STATE OF THE ART

In the field of dyeing of fabrics many problems arise from the hydrophobic nature of the so called 'disperse dyes'. As it is known these dyes are used finely dispersed in water, wherein they have an extremely low solubility, for dyeing polyester, acetate, triacetate, polyamide, polyacrylonitrile, modacrylic and polyvinyl chloride fibres. The two main classes of these compounds are the azo and anthraquinone dyes and can differ considerably in molecular weight.

Since these dyes are not soluble in water, that is the almost exclusive vehicle for dyeing, large amounts of surface agents are added in order to overcome this difficulty which results in high costs both because of the products used (often in very high quantity) and for the huge amount of water requested for washing them out at the end of the process; moreover it must be considered also the problem posed by the risk for the health connected with the use of these products and the costs and difficulties for the treatment of the wastewaters.

It should also be considered that each tinctorial plant uses a particular set of dyes, eventually not similar to that used by another company for the same materials. Also, each dye is used only for a specific fabric. This aspect is well represented from the huge number of dyes on the market, around 10,000 and drives two more problems: the cost of the process is kept high, since each dye is not used in large quantities, and the depuration is difficult and not efficient, since of course cannot be specific. Also reactive dyes present problems, since, just because they are reactive, they react with the water, so that the losses can arrive to 50% of the total dye, increasing the costs and worsening the aspect of depuration.

EP1462484 concerns with new colouring agents comprising a chromophore bound, *via* an ether or ester bond, to one or more molecules of a mono- or disaccharide, the process to prepare them, and their use in the dyeing of textile fibres. These dyes however turned to be soluble in water only when the chromophore molecular weight is below 250.

EP2085434 describes disperse dyes soluble in water of general formula (A)

D-L-Sn (A)

wherein: D is a disperse dye insoluble in water, L is a linker, S is a sugar, n is comprised between 2 and 4.

In particular in EP2085434 are described dyes of formula (I) 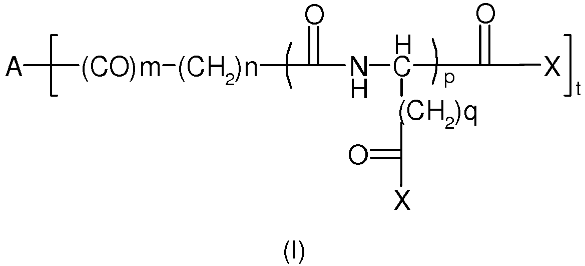 wherein X is 6'-deoxy-6'-amino-lactose; A is a chromophore moiety; p=0, 1 and when p=1 then q=1, 2; n=0, 1, 2, 3; m=0, 1; t=1, 2 proviso that is t=1 when p=1.

Said dyes, in comparison with the corresponding non-glycoconiugated dyes, are able to dye in an homogeneous way natural fibres and textiles and also textile materials including different fibres, and have high biodegradability through digestion by microorganisms, that found in the molecule itself a useful nourishment for their survival and proliferation; processes for the preparation of the above said dyes and their use are also described.

According to the state of the art dyeing of polyester textile is performed at 120-135°C at a pressure higher than atmospheric.

It is therefore evident the importance of making available colouring products having high water solubility at room temperature (also of disperse dyes having high molecular weight). It is also important that said product could be used for dyeing different material (such as various textiles, leather, woods and hair).

### SUMMARY OF THE INVENTION

Subject-matter of the present invention is a compound of formula (I) 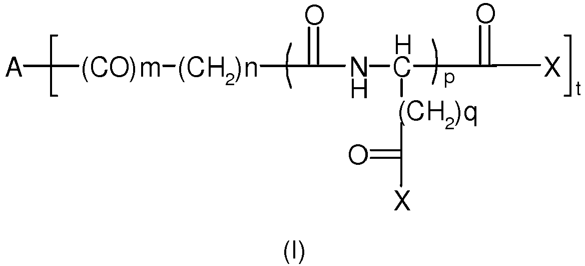 wherein
X is 6'-deoxy-6'-(piperazinyl)lactose
   X = 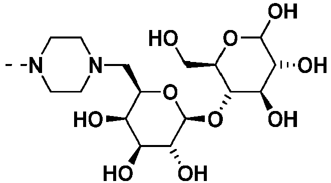
A is a chromophore moiety;
p₌0, 1 and when p=1 then q=1, 2;
n=0, 1, 2, 3;
m=0, 1;
t=1, 2 proviso that is t=1 when p=1;
and wherein all possible optical isomers are included, such as enantiomers and/or diastereoisomers, mixtures thereof, both as racemes or in various ratios, and inorganic or organic salts.

The above compounds are useful as dyeing agents; in particular they are useful for permanent dyeing of leather, wood, textile and for semi-permanent hair dyeing. Subject-matter of the present invention is therefore also a dyeing composition comprising a compound of formula (I) as above described.

Surprisingly the presence of a piperazine unit bonded directly to the saccharidic unit imparts some peculiar and advantageous characteristics:
- the dyes are highly soluble in water, specifically at room temperature;
- the dyes are able to dye hair in a very attractive and useful manner (resistant to washings);
- the dyes are able to dye leather in all tanning conditions, as a matter of fact well resisting even to the most harsh conditions of tanning. The solidity to the washing is at the higher levels ;
- the dyes are able to dye polyester in a temperature range between 95 and 135 °C, preferably at atmospheric pressure and in a pH range from 4 to 10 and in the absence of dyeing auxiliaries;
- the dyes are capable of dyeing wood, displaying wood penetration properties rather than adsorption on the external surface of the material.

For an aspect the present invention relates to a process for preparing a compound of formula (I) as above described, said process comprising the following step:
(b) a deprotection step of the acetonide hydroxy functions of the 6'-deoxy-6'-(piperazinyl)lactose moiety, of a compound of formula (II): 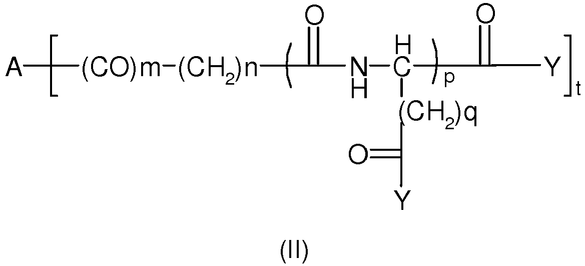 wherein
Y = 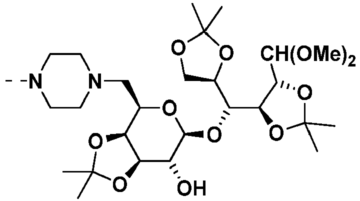 A, p, q, n, m and t are as above defined.

Further subject-matter of the present invention is therefore a compound of formula (II) as above defined, which is key synthetic intermediate for the preparation of a corresponding compound of formula (I) as above defined.

For an aspect subject-matter of the present invention is a method for dyeing textiles, preferably polyester, wherein is used a compound of formula (I) as above described.

Further subject-matter of the present invention is a method for dyeing wood, leather or hair wherein is used a compound a formula (I) 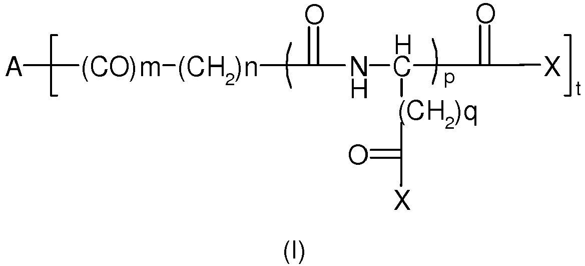 wherein
X is 6'-deoxy-6'-(piperazinyl)lactose or 6'-deoxy-6'-amino-lactose
A is a chromophore moiety;
p=0, 1 and when p=1 then q=1, 2;
n=0, 1, 2, 3;
m=0, 1;
t=1, 2 proviso that is t=1 when p=1;
and wherein all possible optical isomers are included, such as enantiomers and/or diastereoisomers, mixtures thereof, both as racemes or in various ratios, and inorganic or organic salts.

For an aspect subject-matter of the present invention is a method for dyeing polyester at a temperature between 95 and 135 °C, preferably at atmospheric pressure and in pH conditions ranging from 4 to 10 and in the absence of dyeing auxiliaries, said method wherein is used a compound of formula (I) as above described.

### DETAILED DESCRIPTION OF THE INVENTION

The chromophore moiety is preferably selected in the group consisting of diaryl-azoic chromophores, anthraquinone chromophores and aniline chromophores.

The chromophore moiety is bonded to the rest of the molecule through a terminal function selected in the group consisting of a primary or secondary amine, an hydroxy group and a carboxylic function thus giving place to an amino linkage, an ether linkage, an amide linkage or an ester linkage.

Preferably according to the present invention the chromophore moiety is selected in the group consisting of: 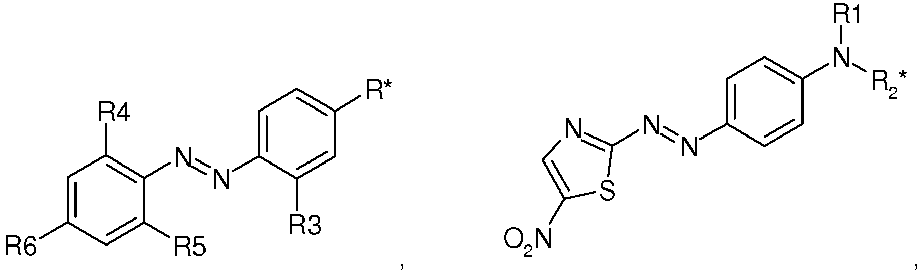 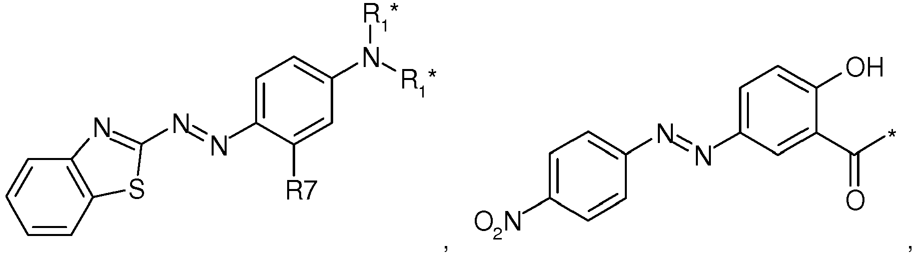 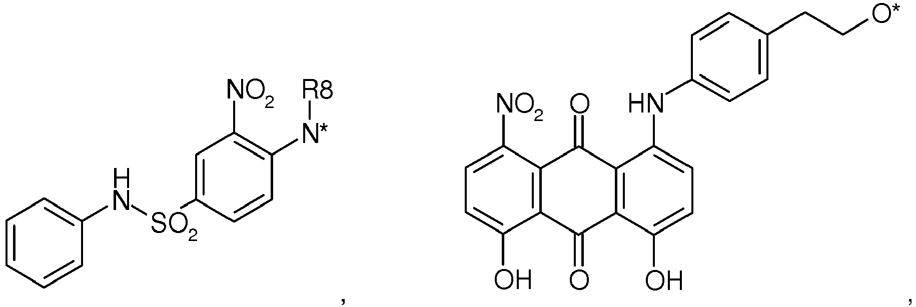 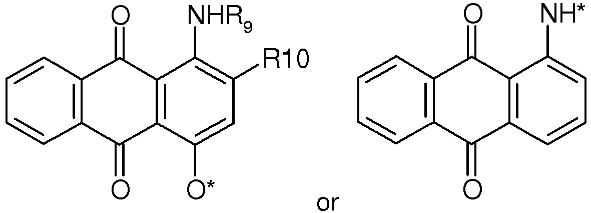
wherein * indicates the point of attachment of the chromophore within the compound of formula (I);
R is N(R1)R2*, CO* or O*;
R1 is CH2CH2CN, CH2CH3, H or R2;
R2 is absent or CH2CH2O*;
N(R1)R2* can also be 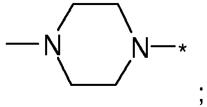
R3 is NHAc, H, Cl or CH3;
R4 is H, Cl or CN;
R5 is H or Cl;
R6 is NO2, F, N(CH3)2 or N(CH2CH2OH)2;
R7 is H or Cl;
R8 is H or Ph;
R9 is H or 4-methyl-phenyl;
R10 is OPh or Br.

According to the invention is preferred a compound of formula (I) wherein p=0 therefore resulting in a compound of formula (III) 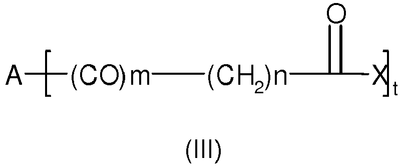 wherein, as said above, n=0, 1, 2, 3; m=0, 1; t=1, 2.

According to the invention within formula (III) are encompassed the following sub-formulae:

| | t=1 | t=2 |
|---|---|---|
| n=0 and m=0 | 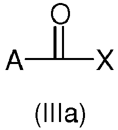 | 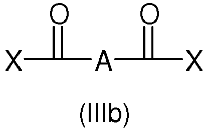 |
| n=1, 2, 3 and m=0 | 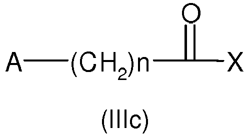 | 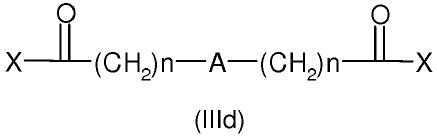 |
| n=0,1,2,3 and m=1 | O O A (CH2)n X (IIIe) | 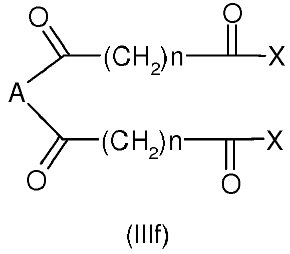 O O (CH2)n X A (CH2)n X O O (IIIf) |

More preferably, in a compound of formula (III), n=2 when m=1.

According to the invention is preferred a compound of formula (I) wherein p=1, then t=1, therefore resulting in a compound of formula (IV): 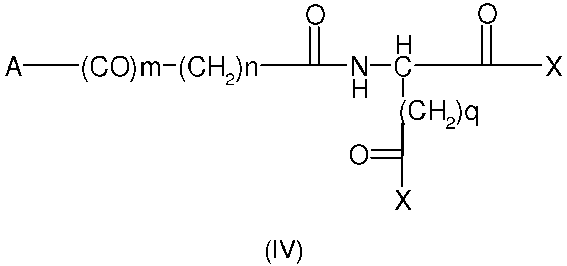 wherein q=1, 2; n=0, 1, 2, 3; m=0, 1.

According to the invention within formula (IV) are encompassed the following sub-formulae:

| | |
|---|---|
| q=1,2; n=0, m=0 | 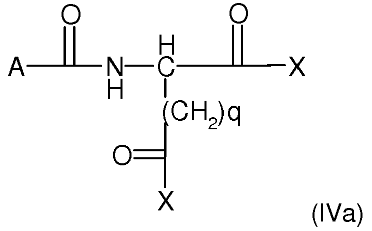 |
| q=1, 2; n=1, 2, 3; m=0 | 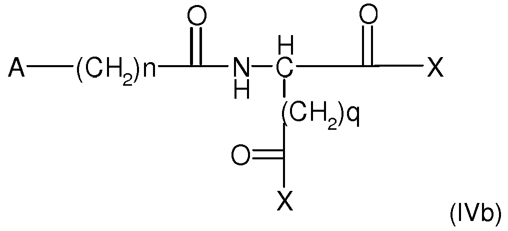 |
| q=1, 2; n=0,1,2,3; m=1 | 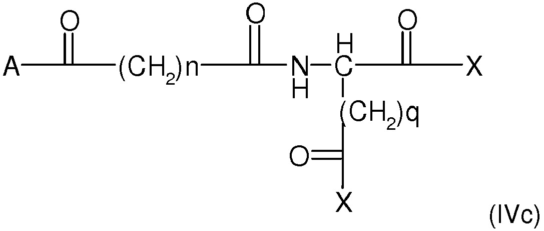 |

More preferably, in a compound of formula (IV), m=1 and n=1.

According to the present invention a compound of formula (I) as above defined, or preferably a compound of formula (III) or (IV) as above defined, can be prepared by a process, as above described, which implies the use of a compound of formula (II) as above defined.

Preferably, according to the present invention, a compound of formula (II) is prepared by a process comprising the following coupling step (a) wherein a compound of formula **1a** 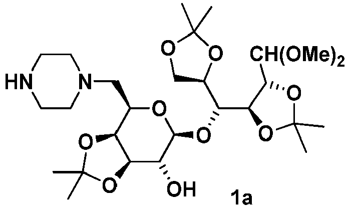 is subjected to a coupling with a compound of formula (V) 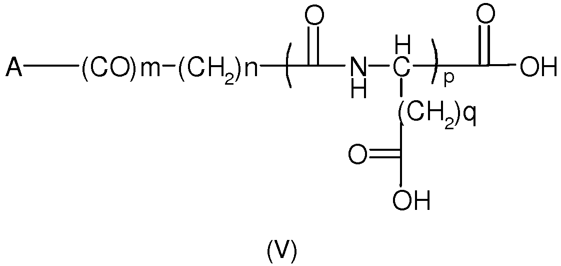 wherein A, p, q, n and m are as above defined and wherein are included possible derivatives of the terminal carboxylic function which can be useful, according to the common general knowledge, in favouring the coupling reaction.

Coupling step (a) is preferred when p=1.

A compound of formula (V) as above defined can be found commercially available (in particular when m=0 and p=0) or otherwise, if not already known in the literature, it can be prepared with well known procedures starting from commercially available compounds and chromophores (as it will be evident from what is described in the experimental section).

Alternatively, and preferably when p=0 and m=1, a compound of formula (II), as above defined, can be prepared by a process comprising the following coupling step (a') wherein a compound of formula (VI) 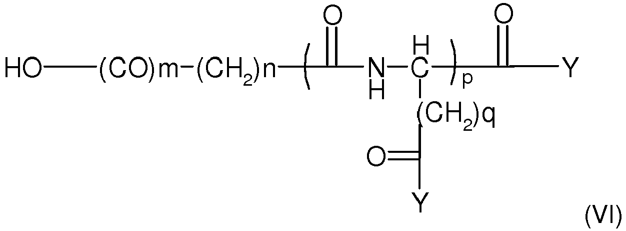 is subjected to a coupling with a compound of formula A-H wherein said H is part of an amino or hydroxy terminal group of the chromophore moiety A;
and wherein Y, A, p, q, n and m are as above defined.

According to the above described processes deprotection step (b) as above described can be preceded by a coupling step (a) or alternatively by a coupling step (a').

Further subject-matter of the present invention is compound **1a** 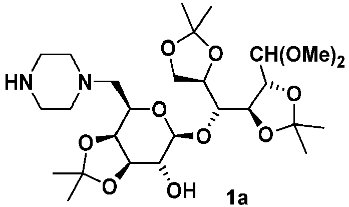 or a compound of formula (VIa): 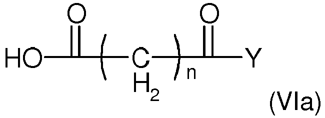 wherein
Y = 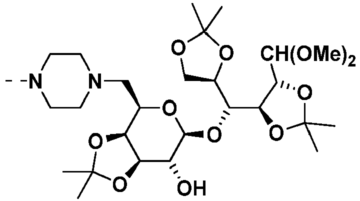 n=0,1,2,3 and wherein are included possible derivatives of the terminal carboxylic function which can be useful in favouring the coupling reaction.

The compounds according to the invention have been used as water soluble colouring agents for dyeing leather, textiles (polyester in particular), wood and hair. Due to their high water solubility the dyeing process is simplified and the resulting colour displayed satisfactory resistance to fastness tests.

The colouring agents of the present invention are the results of chemical transformations of commercially available chromophores or custom designed chromophores, belonging to the category of disperse dyes which are specifically used to dye synthetic textiles and in particular polyester.

One of the main advantages of the compounds of formula (I) as above described is given by the fact that said colouring agents dissolve in water without the need of surfactants, auxiliaries or additives commonly used, or already present within the commercial formulation of disperse dyes, in dyeing protocols for textile fibres, leather and wood. As a matter of fact any attempt to dye textile fibres, leather and wood with a colouring agent wherein a chromophore is chemically bound to piperazine only (lacking the lactose moiety) resulted in colour staining.

Surprisingly it has been found that the compounds according to the present invention are compatible with standard formulations for hair dyeing: that is, said colouring agents can be used as fine powders without the need of developing tailored formulations for hair dyeing.

The colouring agents of the present invention display a multipurpose application character, contrary to the existence of specific dyes designed to dye specifically single materials. Colouring agents according to the present invention demonstrate good affinity for leather, wood, hair and synthetic fibres, specifically polyester, since the resulting hues are homogeneous without local colour spotting or significant colour changes within the dyed sample.

The above said affinity for leather is demonstrated by the ability of the said colouring agent to penetrate the thickness of the leather sample at about 50% depth.

Advantageously the colouring agents according to the present invention display satisfactory levels of fastness on average, when used for dyeing leather as reported in **Table 1:** in particular compound **6b, 17d** and **18c** display excellent level of fastness, which indicate that the said colouring agents are suited for dyeing leather to be used for leather manufacturing goods.

Another advantage is the resistance of the said colouring agents to standard washing protocols when applied to dyed hair as reported in **Table 2.** In addition, said colouring agents fade linearly with the increase number of washings applied to the dyed hair. The above said fading according to the number of washings, is consistent with the colour change due to the new growing hair: that is, the said colouring agents are defined substantive and suited to dye hair in a semi-permanent fashion.

A further ability of the colouring agents according to the present invention is displayed when used for dyeing wood, into which the dye is able to penetrate at about 0.5 mm depth instead of being adsorbed purely on the external surface of the wood sample. In addition, the light fastness of the colouring agents of the invention when used for dyeing wood is of good level as reported in **Table 3:** that is, the observed light fastness is even above that one observed on average for dyed leather and polyester samples with the colouring agents of the present invention.

Further advantage of the present invention is the fact that the above said affinity for synthetic fibres, specifically polyester, is demonstrated by the excellent level of fastness reported in **Table 4.** In particular, the colouring agents display excellent level of fastness to solvent and sublimation: that is, the tendency to colour change or colour migration due to the high level of dispersion of commercial disperse dyes on polyester, is prevented by the modification brought by the present invention to chromophores belonging to the class of disperse dyes.

The chromophore of the compounds of the present invention have been taken from disperse dyes for dyeing synthetic fibres; said chromophore, once chemically naturalised with the 6'-deoxy-6'-(piperazinyl)lactose moiety, become able to dye not only synthetic fibres but surprisingly leather, hair and wood. Furthermore, with a compound of the present invention polyester can be surprisingly, dyed even at temperature below 100 °C and preferably at atmospheric pressure. Said dyeing process can be carried out in a wide range of pH, between 4 and 10, preferring pH values between 8 and 10. Advantageously, by virtue of the high water solubility of the compounds of the present invention, said process can be performed in the absence of dyeing auxiliaries, commonly used to obtain hue homogeneity. In the above said conditions the fastness properties of the colored fiber improve, making unnecessary common processes for cleaning textiles (stripping) using sodium bisulfite after the dyeing step. The resulting hues of the colouring agents of the invention may appear different, in some cases and depending from the dyed material, to the colour tones or shades of the declared hues (from suppliers within the colour trade or from the Colour Index) on the synthetic textile fibres normally dyed with commercial disperse dyes which have been used as chromophore. As clarifying examples, compounds **7b** and **8b** results in pink hue on leather instead of maintaining the declared bright red colour of the parent chromophore Disperse Red 60 when used for synthetic textile fibres; compound **17d** moves from brilliant yellow of the parent Disperse Yellow 42 to a yellow-green hue on leather; compound **19e** changes from red-light blue of the parent chromophore Disperse Blue 72 to light violet on leather as a result of the chemical transformation on the chromophore structure.

As a confirmation of different hues expected on leather for the said colouring agents coming from chromophores belonging to the class of disperse dyes designed for textile dyeing, is the fact that compound **2d** appears red when dissolved in water, whilst displaying a deep orange colour when used in dyeing leather in contrast to a red colour declared for the parent compound Disperse Red 54 when used for dyeing synthetic textile fibres; compound **4b** shows a brown shade on leather whereas an aq. solution is red and consistent with the declared color of the parent chromophore Disperse Red 202; or compound **14b** displays a bright red colour in water whilst being orange when used in dyeing leather.

The said above considerations for the resulting hues on leather may well apply to the said colouring agents when used for dyeing wood and hair and polyester fabrics, according to the present invention, which may promote different penetration/adsorption modes of the dye within the polyester fabric.

The present invention can be better understood in light of the following examples.

### EXPERIMENTAL SECTION

### References

1. W. C. Still, M. Kahn, A. Mitra, J. Org. Chem. 1978, 43, 2923-2925.
2. R. Bianchini, M. Rolla, J. Isaad, M. Corsi, M. Bonanni, G. Catelani, L. Guazzelli, Carbohydr. Res. 2012, 356, 104-109.
3. G. J. Ewing, K. B. Mullah, R. J. Graham, US 2003/0082547 A1.
4. J. A. Kiritsy, D. K. Yung, J. Med. Chem. 1978, 21, 1301-1307.
5. P. W. Barker, V. Boyd, B. R. Fishwick, Brian R., M. Roche, 1974 DE 2339984 A1T.
6. Hu, H. Xie, L. Chen, S. Chen, H. Zhang, Polym. Bull. 2011, 67, 937-950.
7. R. Bianchini, G. Catelani, F. D'Andrea, J. Isaad, M. Rolla, F. Bonaccorsi, T. Nocentini, 2009 EP2085434 A1.
8. Y. C. Chao, S. M. Lin, Dyes and Pigments 1998, 37, 357-371.
9. M. Kunishima, C. Kawachi, F. Iwasaki, K. Terao, S. Tani, Tetrahedron Lett. 1999, 40, 5327-5330.

### Materials and analytical methods.

Commercially available reagents and solvents were purchased from SigmaAldrich and they were used directly unless otherwise specified. Thin layer chromatography (TLC) analysis was performed using Fluka aluminum foils coated with 25 mm particle size silica gel matrix F254. TLC development involved either UV (254 and 366 nm) or visible light inspection, followed by either treatment with an acid solution of *p*-anisaldehyde or a basic solution of KMnO₄ and heating. Flash column chromatography was performed on Merck silica gel 60 (particle size 0.040-0.063 nm, 230-400 mesh ASTM) according to the procedure of Still.¹ Melting points were recorded on a Melting Point Apparatus SMP3-STUART SCIENTIFIC. UV-Vis spectra were recorded on a Cary-4000 Varian spectrophotometer, using 1 cm quartz cuvettes. ¹H-NMR spectra were recorded at either 200 or 300 MHz on a Varian Gemini spectrometer. Spin resonances were reported as chemical shifts (*δ*) in parts per million (ppm) and referenced to the residual peak as an internal standard of the solvent employed, as follows: CDCl₃ 7.27 ppm, DMSO-d₆ 2.51 ppm. Spin multiplicity was indicated by s=singlet, d=doublet, t=triplet, q=quartet, qn=quintet, m=multiplet, br=broad. Coupling constants *J* were reported in Hertz. Mass spectra were recorded on a ThermoScientific LCQ-Fleet mass spectrometer under electrospray ionisation (ESI, +c or -c technique). Mass spectrometric analyses were quoted in the m/z form.

### Colour fastness tests

Light fastness tests were carried out exposing dyed leather and polyester fabric samples to the light radiation of a Xenon lamp for 48 and 8 h respectively. The blue wool fabric scale from 1 (low resistance) to 8 (high resistance) was used to assess the results of light fastness tests.

Migration tests to PVC for leather were carried out keeping wet dyed leather samples in contact with a 1 mm thick PVC specimen for 2 h at 50 °C under pressure.

Staining fastness to rubbing for leather was carried out using a piece of standard water wet wool felt under a given pressure with twenty forward and backward motions.

Decolorization induced by washing for polyester fabric was carried out in a washing drum at 60 °C for 0.5 h and in the presence of 25 small stainless balls.

Staining fastness to rubbing for dyed polyester fabric was carried out both in dry and wet conditions, stroking the fabric ten times with a 1.1 Kg mass stroke carrier.

Sublimation tests for polyester fabrics were carried out at 180 °C for 0.5 h, keeping the fabric in contact with a white cotton specimen to assess color migration.

Decolorization induced by washing for dyed hair was carried out using a shampoo of the Salone type and tap water, with 3, 6, 9 and 12 wash cycles.

The standard grey scale from 1 (high tendency) to 5 (low tendency) was used to assess the results of fastness tests to stain and to decolorization.

### Literature procedures of some known compounds.

Compound **1** was synthesized according to the procedure of Bianchini *et al..*² 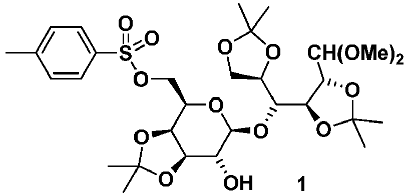
Compound **2** was synthesized according to the procedure of Ewing *et al..*³ 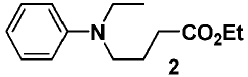
Compound **3** was synthesized according to the procedure of Kiritsy *et al..*⁴ 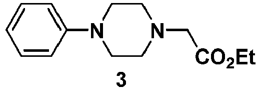
Compound **4** was synthesised according to the procedure of Barker *et al..*⁵ 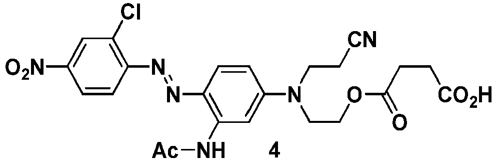
Compound **5** was synthesized according to the procedure of Bianchini *et al..*² 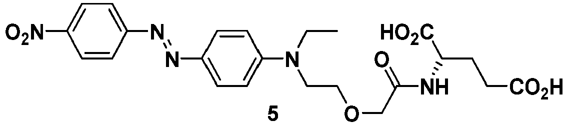
Compound **6** was synthesized according to procedure of Hu *et al..*⁶ 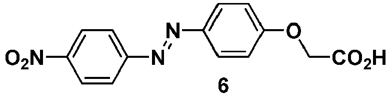
Compound **7** and **8** were synthesized according to the procedure of Bianchini *et al..*⁷ 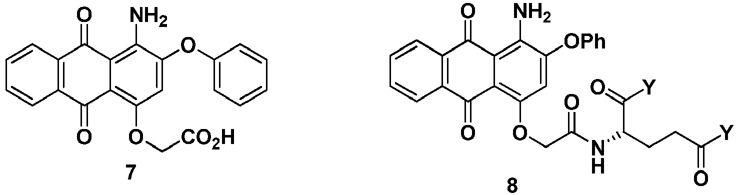
Compound **9** was synthesized according to the method of Chao et al..⁸ 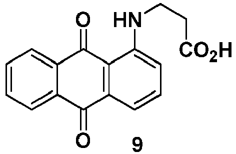
Compound **10** was synthesized according to the procedure of Kunishima *et al..*⁹

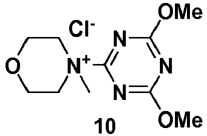

### General synthetic procedures

### Isolation of dyes from commercial preparations.

**Method A:** A commercially available preparation of a dye for tinctorial purposes was slurried in dichloromethane [1:10 ratio (w/v)] at 20 °C for 2 h. The mixture was passed through a filter, made of a layer of paper, cotton and sand, under suction. The filtrate was concentrated to dryness, to recover the dye as fine powder.

### Synthesis of diaryl-azo dye species.

**Method B:** A suspension of the appropriate primary aromatic amine (1 mmol) in aq. hydrochloric acid was aged at 20 °C for 2 h and then it was treated with an ice-cooled solution of sodium nitrite (1 mmol, 20% w/w in water) at 0 °C. The resulting diazonium salt containing mixture was stirred for a further 0.5 h and a solution of the appropriate tertiary aromatic amine (1 mmol) dissolved in acid aq. ethanol was added dropwise. The resulting deep coloured slurry was warmed to 20 °C over 1 h. The mixture was diluted with water and an appropriate base was added to neutralize. The fine slurry was further aged for 2 h and then it was filtered under suction. The press cake was washed thoroughly with water and dried in air.

**Method C:** A suspension of the appropriate primary aromatic amine (1 mmol) in acetic acid (2.1 mL/mmol) and propionic acid (0.4 mL/mmol) was cooled to 0 °C and treated with a solution of nitrous sulfuric anhydride (1 mmol, 40% w/w in conc. sulphuric acid). The resulting solution was stirred for 0.5 h and it was added dropwise to a slurry of an appropriate tertiary aromatic amine (1 mmol) in ethanol (5 mL/mmol) and ice (5 g/mmol). The resulting deep coloured mixture was stirred for 1 h at 0 °C: then, it was diluted with water and warmed to 20 °C overnight. The fine suspension was filtered under suction and the press cake was washed thoroughly with water and dried in air.

### Hydrolysis of carboxylic ester derived dyes.

**Method D:** A solution of the appropriate ester derivative (1 mmol) was dissolved in tetrahydrofuran (5-10 vol/g) and treated with 1 M aq. potassium hydroxide (1.5-2.0 mmol/mmol) at 0 °C. The resulting mixture was stirred for 0.3 h and then it was warmed to 20 °C overnight. Aq. 1 M hydrochloric acid was added to bring the pH to 2-3 and the fine slurry was filtered under suction. The press cake was washed with a cold mixture of methanol - water (1:10, v/v) and dried in air.

### Synthesis of 4-oxobutanoic acid carboxylic ester dye derivatives.

**Method E:** An ice-cooled solution of the appropriate dye (1 mmol) dihydrofuran-2,5-dione (1.1-1.2 mmol) and *N,N*-dimethylpyridin-4-amine (0.1 mmol) in either dichloromethane or tetrahydrofuran (4-5 mL/mmol) was treated with triethylamine (1.0-1.3 mmol) at 0 °C dropwise. The resulting mixture was warmed to 20 °C overnight: then, acetic acid (0.3 g/mmol) was added and the whole was stirred for 1 h. The solution was washed with water and brine: the organic phase was then separated, dried over anhydrous sodium sulphate and filtered. The filtrate was concentrated *in vacuo* to yield the desired compound as coarse solid, which was dried in air.

### Synthesis of amide derivatives of dyes.

**Method F:** A solution of the appropriate carboxylic acid dye (1 mmol) in either anhydrous dichloromethane or tetrahydrofuran (3.9-4.5 mL/mmol) under a nitrogen atmosphere was cooled to 0 °C. Sulfurous dichloride (1.1 mmol) was added dropwise and the resulting mixture was stirred for 0.3 h: then, it was warmed to 20 °C in 1 h and it was finally added to an ice-cooled solution of compound **1a** (1.0 mmol) and triethylamine (3.0 mmol) in dichloromethane (3.9-4.5 mL/mmol). The whole was kept at 0 °C for 0.3 h, warmed to 20 °C and washed with water, aq. sat. sodium hydrogencarbonate, water and brine. The organic phase was separated, dried over anhydrous sodium sulphate and filtered. The filtrate was concentrated under reduced pressure to yield the desired compound as foamy colored solid.

**Method G:** A solution of the appropriate carboxylic acid dye (1 mmol) and compound **1a** (1.1 mmol) in anhydrous tetrahydrofuran (10-15 mL/mmol) was stirred for 4 h at 20 °C under a nitrogen atmosphere. Compound **10** (1.1 mmol) was added portionwise and the resulting mixture was stirred overnight. The solvent was evaporated *in vacuo* and the residue was partitioned between ethyl acetate and water. The organic phase was separated, washed with brine, dried over anhydrous sodium sulphate and filtered. The filtrate was concentrated under reduced pressure, to yield the desired compound as foamy colored solid.

**Method H:** A solution of the appropriate carboxylic acid dye (1 mmol), (S)-1,5-diethoxy-1,5-dioxopentan-2-aminium chloride (1.1 mmol) and 4-methylmorpholine (1mmol) in anhydrous tetrahydrofuran (12-15 mL/mmol) was stirred for 4 h at 20 °C under a nitrogen atmosphere. Then, compound **10** (1.1 mmol) was added and the procedure described in "Method G" followed next.

### Deprotection of 2,2-dimethyl-1,3-dioxolane functional groups of carboxylic acid dye derivatives.

**Method I:** A solution of the appropriate protected carboxylic acid dye derivative (1 mmol) in acetone (10-12 mL/mmol) was cooled to 0 °C and an ice-cooled solution of aq. hydrochloric acid (2.5-3.0 mmol, 37% w/w) in acetone (0.7-1.0 mL/mmol) was added dropwise. The resulting mixture was warmed to 20 °C overnight, after which the solvent was decanted. The precipitated residue was slurried in fresh acetone three times and then it was dried *in vacuo* to fine colored powder.

**Method J:** A solution of the appropriate protected carboxylic acid dye derivative (1 mmol) was dissolved in 90% aq. (v/v) 2,2,2-trifluoroacetic acid (20 mL/g) at 0 °C. The mixture was stirred for 0.3 h: then, it was warmed to 20 °C over 2 h. The solvent was evaporated under reduced pressure and the crude was repeatedly coevaporated with toluene *in vacuo.* The residue was slurried in acetone, the solvent was decanted and the desired product was isolated and dried *in vacuo* as fine colored powder.

### 4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazine 1a.

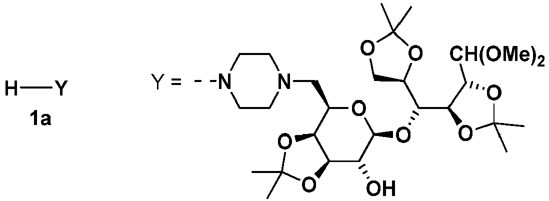

In a three neck round bottomed flask equipped with a condenser, a thermometer and a dropping funnel, piperazine (63.7 g, 739 mmol) was suspended in acetonitrile (540 mL) under nitrogen. The mixture was brought to reflux and a solution of compound **1** (49.0 g, 73.9 mmol) in acetonitrile (60 mL) was added dropwise over 1 h time. Reflux was maintained for further 6 h, after which the solution was cooled to 20 °C. Acetonitrile was evaporated under reduced pressure and the residue was recovered with dichloromethane and washed with water to eliminate the excess of piperazine. The organic phase was dried over anhydrous sodium sulphate, filtered and evaporated to yield the title compound (40.2 g, 94%) as off white foamy solid, mp 62-64 °C; *δ*_{H} (300 MHz, CDCl₃): 4.42-4.32 (3H, m), 4.27 (1H, td, *J* 2.4 and *J*' 6.6 Hz), 4.17-3.99 (5H, m), 3.90 (1H, dd, *J* 1.5 and *J*' 7.5 Hz), 3.83 (1H, td, *J* 2.1 and *J*' 6.0 Hz), 3.56-3.51 (1H, m), 3.46-3.42 (1H, m), 3.45 (3H, s), 3.44 (3H, s), 2.89 (4H, t, *J* 4.8 Hz), 2.80-2.64 (2H, m), 2.61-2.46 (4H, m), 2.37-2.07 (1H, br), 1.51 (3H, s), 1.49 (3H, s), 1.39 (3H, s), 1.37 (3H, s), 1.34 (3H, s), 1.33 (3H, s); ESI (m/z, +c): 577.6 (100%) [M+H]⁺.

### 4-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanoic acid 1b.

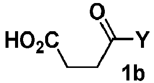

In a one neck round bottomed flask compound **1a** (16.3 g, 28.2 mmol) was dissolved in dichloromethane and dihydrofuran-2,5-dione (2.82 g, 28.2 mmol) was added at 20 °C. The solution was stirred for 2 h and then, the solvent was evaporated under reduced pressure to obtain compound **1b** as off white powder, mp 74-76 °C; *δ*_{H} (300 MHz, CDCl₃): 4.45-4.24 (4H, m), 4.19-3.99 (5H, m), 3.92-3.89 (2H, m), 3.74-3.40 (5H, m), 3.47 (3H, s), 3.46 (3H, s), 2.91-2.42 (10H, m), 1.50 [6H, s (br)], 1.39 (3H, s), 1.38 (3H, s), 1.34 (3H, s), 1.33 (3H, s); ESI (m/z, +c): 677.4 (100%) [M+H]⁺, 699.5 (20%) [M+Na]⁺.

### (E)-Ethyl 4-{ethyl-{4-[(4-nitrophenyl)diazenyl]phenyl}amino}butanoate 2a.

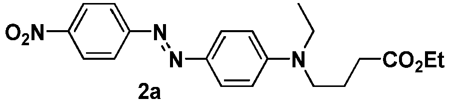

The synthesis of compound **2a** was carried out on 12.0 mmol scale of 4-nitroaniline (1.66 g) suspended in 6 M aq. hydrochloric acid (1.0 mL) using sodium nitrite (0.83 g, 12.0 mmol, aq. 20% w/w), compound **2** (2.82 g, 12.0 mmol), ethanol (3.5 mL) made acid with 6 M aq. hydrochloric acid (2.4 mL), water (100 mL) and an aq. 1 M solution of sodium hydroxide to neutralize, according to "Method B". Compound **2a** (4.18 g, 90.7%) was obtained as dark red solid, mp 93-95.5 °C; *δ*_{H} (300 MHz, CDCl₃): 8.33 (2H, d, *J* 9.3 Hz), 7.92 (2H, d, *J* 9.3 Hz), 7.90 (2H, d, *J* 9.3 Hz), 6.78 (2H, d, *J* 9.3 Hz), 4.18 (2H, q, *J* 7.0 Hz), 3.54-3.43 (4H, m), 2.41 (2H, t, *J* 7.0 Hz), 2.00 (2H, qn, *J* 7.5 Hz), 1.29 (3H, t, *J* 7.0 Hz), 1.25 (3H, t, *J* 7.0 Hz); ESI (m/z, +c): 385.2 (30%) [M+H]⁺, 791.1 (100%) [2M+Na]⁺.

### (E)-4-{Ethy)-{4-[(4-nitrophenyl)diazenyl]phenyl}amino}butanoic acid 2b.

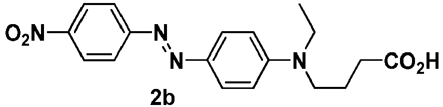

The synthesis of compound **2b** was carried out on 10.4 mmol scale of compound **2a** (4.0 g) using tetrahydrofuran (40 mL), an aq. 1 M solution of potassium hydroxide (1.17 g, 20.8 mmol) and 1 M hydrochloric acid according to "Method D". Compound **2b** (3.63 g, 98.0%) was obtained as dark red solid, mp 168-171 °C; *δ*_{H} (200 MHz, CDCl₃): 8.07 (2H, d, *J* 9.0 Hz), 7.70-7.61 (4H, m), 6.56 (2H, d, *J* 9.4 Hz), 3.34-3.17 (4H, m), 2.14 (2H, t, *J* 7.3 Hz), 1.72 (2H, qn, *J* 7.3 Hz), 1.00 (3H, t, *J* 7.0 Hz); ESI (m/z, -c): 355.6 (24%) [M-H]⁻, 711.5 (100%) [2M-H]⁻.

### 1-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-{ethyl-{4-[(E)-(4-nitrophenyl)diazenyl]phenyl}amino}butan-1-one 2c.

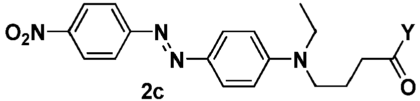

The synthesis of compound **2c** was carried out on 1.0 mmol scale of species **2b** (0.36 g) using anhydrous tetrahydrofuran (10 mL), compound **1a** (0.63 g, 1.1 mmol) and **10** (0.30 g, 1.1 mmol) according to "Method G". Compound **2c** (0.89 g, 97.2%) was obtained as foamy red solid, mp 84.5-86 °C; *δ*_{H} (200 MHz, CDCl₃): 8.32 (2H, d, *J* 9.0 Hz), 7.94-7.84 (4H, m), 6.80 (2H, d, *J* 9.4 Hz), 4.46-4.24 (4H, m), 4.19-4.01 (5H, m), 3.95-3.79 (4H, m), 3.74-3.37 (9H, m), 3.46 [6H, s (br)], 2.82-2.68 (2H, m), 2.66-2.46 (3H, m), 2.45-2.31 (2H, m), 2.05-1.93 (2H, m), 1.50 [6H, s (br)], 1.40 (3H, s), 1.38 (3H, s), 1.35 (3H, s), 1.33 (3H, s), 1.25 (3H, t, *J* 7.4 Hz); ESI (m/z, +c): 915.6 (100%) [M+H]⁺.

### 1-[4-(6'-Deoxy)(lactose-6'-yl)piperazin-1-yl]-4-{ethyl-{4-[(E)-(4-nitrophenyl)-diazenyl]phenyl}amino}butan-1-one hydrochloride 2d.

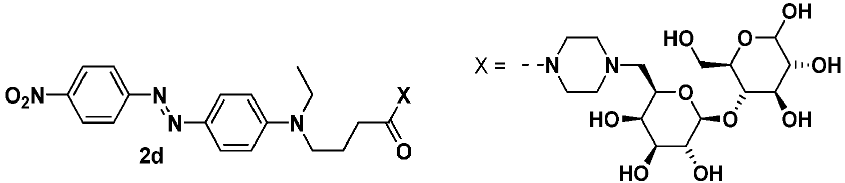

The synthesis of compound **2d** was carried out on 0.87 mmol scale of species **2c** (0.8 g) using acetone (8 mL) and hydrochloric acid (0.22 mL, 37% w/w, 2.6 mmol) dissolved in acetone (1.0 mL), according to "Method I". Compound **2d** (0.62 g, 90.8%) was obtained as foamy dark red solid, mp 124-148 °C (dec.); *λ*ₘₐₓ (ε) (H₂O): 508 nm (23200 M⁻¹cm⁻¹); *δ*_{H} (200 MHz, CDCl₃): 8.37 (2H, d, *J* 8.60 Hz), 7.94 (2H, d, *J* 8.6 Hz), 7.87 (2H, d, *J* 8.60 Hz), 6.97 (2H, d, *J* 8.3 Hz), 5.23-4.23 [17H, m (br)], 4.21-2.78 [20H, m (br)], 1.18 [3H, s (br)]; ESI (m/z, +c): 749.4 (100%) [M+H]⁺.

### (E)-Ethyl 2-{4-{4-[(4-nitrophenyl)diazenyl]phenyl}piperazin-1-yl} acetate 3a.

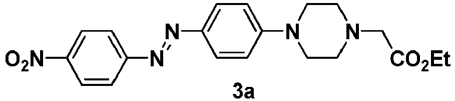

The synthesis of compound **3a** was carried out on 5.0 mmol scale of 4-nitroaniline (0.69 g) suspended in 6 M aq. hydrochloric acid (4.5 mL) using sodium nitrite (0.35 g, 5.0 mmol, aq. 20% w/w), compound **3** (1.24 g, 5.0 mmol), ethanol (3 mL) made acid with 6 M aq. hydrochloric acid (1.1 mL), water (40 mL) and an aq. 1 M solution of sodium hydroxide to neutralize, according to "Method B". Compound **3a** (1.45 g, 73.2%) was obtained as dark red solid, mp 120-121 °C; *δ*_{H} (300 MHz, CDCl₃): 8.34 (2H, d, *J* 9.2 Hz), 7.97-7.90 (4H, m), 6.97 (2H, d, *J* 9.2 Hz), 7.08 (2H, d, *J* 9.3 Hz), 4.23 (2H, q, *J* 7.2 Hz), 3.53-3.46 (4H, m), 3.30 (2H, s), 2.81-2.73 (4H, m), 1.30 (3H, t, *J* 7.2 Hz); ESI (m/z, +c): 398.3 (100%) [M+H]⁺.

### (E)-2-{4-{4-[(4-Nitrophenyl)diazenyl]phenyl}piperazin-1-yl}acetic acid 3b.

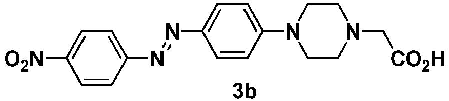

The synthesis of compound **3b** was carried out on 3.52 mmol scale of compound **3a** (1.40 g) using tetrahydrofuran (15 mL), an aq. 1 M solution of potassium hydroxide (0.4 g, 7.04 mmol) and 1 M hydrochloric acid according to "Method D". Compound **3b** (1.1 g, 85.0%) was obtained as dark red solid, mp 215-217 °C; *δ*_{H} (300 MHz, DMSO-d₆): 8.39 (2H, d, *J* 9.0 Hz), 7.98 (2H, d, *J* 9.0 Hz), 7.87 (2H, d, *J* 9.0 Hz), 7.13 (2H, d, *J* 9.0 Hz), 3.56-3.49 (4H, m), 3.26 (2H, s), 2.78-2.68 (4H, m); ESI (m/z, +c): 370.3 (100%) [M+H]⁺.

### 1-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-2-{4-{4-[(E)-(4-nitrophenyl)diazenyl]phenyl}piperazin-1-yl}ethanone 3c.

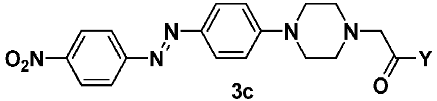

The synthesis of compound **3c** was carried out on 2.0 mmol scale of species **3b** (0.74 g) using anhydrous tetrahydrofuran (30 mL), compound **1a** (1.15 g, 2.2 mmol) and **10** (0.61 g, 2.2 mmol) according to "Method G". Compound **3c** (1.56 g, 84.3%) was obtained as foamy orange-red solid; *δ*_{H} (300 MHz, CDCl₃): 8.34 (2H, d, *J* 9.3 Hz), 8.00-7.91 (4H, m), 6.97 (2H, d, *J* 9.3 Hz), 8.00 (1H, d, *J* 9.0 Hz), 6.98 (2H, d, *J* 9.3 Hz), 4.45-4.23 (4H, m), 4.21-3.99 (5H, m), 3.98-3.81 (5H, m), 3.79-3.51 (6H, m), 3.49-3.30 (8H, m), 3.25 (1H, s), 2.78-2.38 (10H, m), 1.51 (3H, s), 1.50 (3H, s), 1.40 (3H, s), 1.38 (3H, s), 1.35 (3H, s), 1.34 (3H, s).

### 1-[4-(6'-Deoxy)(lactose-6'-yl)]piperazin-1-yl}-2-{4-{4-[(E)-(4-nitrophenyl)diazenyl]phenyl}piperazin-1-yl}ethanone hydrochloride 3d.

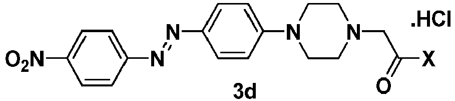

The synthesis of compound **3d** was carried out on 1.51 mmol scale of species **3c** (1.40 g) using acetone (20 mL) and hydrochloric acid (0.38 mL, 37% w/w, 4.53 mmol) dissolved in acetone (1.5 mL) according to "Method I". Compound **3d** (1.07 g, 88.4%) was obtained as dark orange-red solid, mp 330-360 °C (dec.); *λ*ₘₐₓ (ε) (H₂O): 416 nm (13500 M⁻¹cm⁻¹); *δ*_{H} (300 MHz, DMSO-d₆): 8.40 (2H, d, *J* 9.0 Hz), 8.00 (2H, d, *J* 9.0 Hz), 7.92 (2H, d, *J* 9.0 Hz), 7.21 (2H, d, *J* 9.0 Hz), 5.28-4.28 [15H, m (br)], 4.23-4.02 (2H, m), 4.00-3.82 (3H, m), 3.81-3.557 (6H, m), 3.55-2.86 (14H, m); ESI (m/z, +c): 762.5 (100%) [M+H]⁺.

### 2-{{3-Acetamido-4-[(E)-(2-chloro-4-nitrophenyl)diazenyl]phenyl}-[2-cyanoethy l]amino}ethyl 4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanoate 4a.

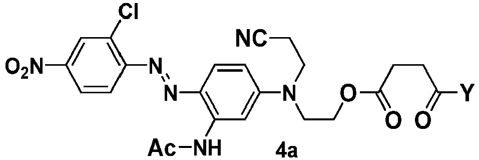

The synthesis of compound **4a** was carried out on 15.0 mmol scale of species **4** (7.96 g) using anhydrous tetrahydrofuran (190 mL), compound **1a** (9.52 g, 16.5 mmol) and **10** (4.57 g, 16.5 mmol) according to "Method G". Compound **4a** (15.1 g, 92.4%) was obtained as foamy red-orange solid; *δ*_{H} (300 MHz, CDCl₃): 8.42 (1H, d, *J* 2.4 Hz), 8.33 [1H, d (br), *J* 2.7 Hz], 8.20 (1H, dd, *J* 2.4 and *J'* 9.0 Hz), 7.90 (1H, d, *J* 9.0 Hz), 7.88 (1H, d, *J* 9.0 Hz), 6.66 (1H, dd, *J* 9.0 and *J'* 2.7 Hz), 4.42-4.24 (6H, m), 4.17-4.0 (5H, m), 3.97-3.82 (8H, m), 3.67-3.52 (3H, m), 3.48-3.42 (2H, m), 3.46 (3H, s), 3.45 (3H, s), 2.83 (2H, t, *J* 4.6 Hz), 2.77-2.72 (1H, m), 2.68-2.42 (8H, m), 2.31 (3H, s), 1.51 (3H, s), 1.49 (3H, s), 1.39 (3H, s), 1.37 (3H, s), 1.34 (3H, s), 1.31 (3H, s).

### 2-{{3-Acetamido-4-[(E)-(2-chloro-4-nitrophenyl)diazenyl]phenyl}-[2-cyano-ethyl]amino}ethyl 4-[4-(6'-deoxy)(lactose-6'-yl)piperazin-1-yl]-4-oxobutanoate hydrochloride 4b.

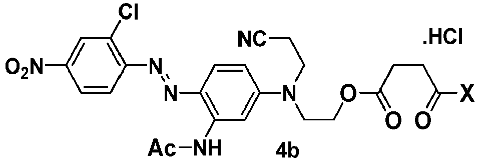

The synthesis of compound **4b** was carried out on 4.22 mmol scale of species **4a** (4.6 g) using acetone (45 mL) and hydrochloric acid (0.87 mL, 37% w/w, 10.5 mmol) dissolved in acetone (10.5 mL) according to "Method I". Compound **4b** (3.47 g, 85.7%) was obtained as dark red solid, mp 170-177 °C (dec.); *λ*ₘₐₓ (ε) (H₂O): 425 nm (12900 M⁻¹cm⁻¹); *δ*_{H} (300 MHz, DMSO-d₆): 11.05 (1H, s), 8.44 (1H, d, *J* 2.7 Hz), 8.25 (1H, dd, *J* 2.4 and *J*' 9.0 Hz), 8.05 (1H, d, *J* 9.0 Hz), 7.99 [1H, d (br), *J* 2.7 Hz], 7.79 (1H, d, *J* 9.0 Hz), 6.87 (1H, dd, *J* 9.0 and *J*' 2.4 Hz), 4.93-4.22 (6H, m), 4.14-3.54 (7H, m), 3.53-3.39 (5H, m), 3.38-3.36 (4H, m), 3.36-2.96 (12H, m), 2.92-2.87 (3H, m), 2.72-2.54 (2H, m), 2.53-2.40 (3H, m), 2.24 (3H, s); ESI (m/z, +c): 923.4 (100%) [M+H]⁺, 925.3 (40%) [M+H]⁺.

### N-{(S)-1,5-Bis-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-1,5-dioxopentan-2-yl}-2-{2-{ethyl-{4-[(E)-(4-nitrophenyl)diazenyl]-phenyl}amino}ethoxy}acetamide 5a.

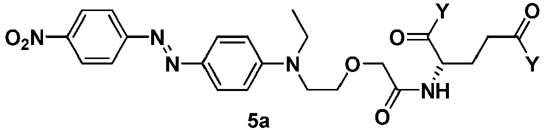

The synthesis of compound **5a** was carried out on 0.60 mmol scale of species **5** (0.3 g) using anhydrous tetrahydrofuran (8 mL), compound **1a** (0.76 g, 1.32 mmol) and **10** (0.37 g, 1.32 mmol) according to "Method G". Compound **5a** (0.92 g, 94.8%) was obtained as foamy dark red solid, mp 90-92 °C; *δ*_{H} (300 MHz, CDCl₃): 8.38-8.28 (2H, m), 7.97-7.86 (4H, m), 7.53-7.44 [1H, m (br)], 6.86-6.75 (2H, m), 6.07-4.92 (1H, m), 4.48-4.26 [7H, m (br)], 4.17-3.96 (18H, m), 3.89-3.70 (14H, m), 3.63-3.29 (21H, m), 2.83-2.10 [8H, m (br)], 1.71 [2H, s (br)], 1.51 [12H, s (br)], 1.39-1.34 (24H, m), 1.26 (3H, t, *J* 6.6 Hz). ESI (m/z, +c): 810.51 (100%) [M+2H]²⁺, 821.6 (10%) [M+H+Na]²⁺, 1618.9 (14%) [M+H]⁺, 1640.9 (8%) [M+Na]⁺.

### N-{(S)-1,5-Bis-[4-(6'-deoxy)(lactose-6'-yl)piperazin-1-yl]-1,5-dioxopentan-2-yl}-2-{2-{ethyl-{4-[(E)-(4-nitrophenyl)diazenyl]phenyl}amino}ethoxy}acetamide ditrifluoroacetate 5b.

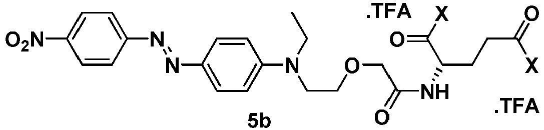

The synthesis of compound **5b** was carried out on 0.56 mmol scale of species **5a** (0.9 g) using 90% aq. (v/v) 2,2,2-trifluoroacetic acid (18 mL), toluene and acetone, according to "Method J". Compound **5b** (0.77 g, 90.6%) was obtained as red solid, mp 108-122 °C (dec.); *λ*ₘₐₓ (ε) (H₂O): 500 (19900 M⁻¹cm⁻¹); *δ*_{H} (200 MHz, DMSO-d₆): 8.38-8.29 (2H, m), 7.96-7.81 (4H, m), 7.23-7.13 (2H, m), 6.95-6.86 (1H, m), 6.00-5.00 [23H, m (br)], 4.96-4.18 (11H, m), 4.08-2.80 (37H, m), 1.15 (3H, t, *J* 6.8 Hz); ESI (m/z, +c): 644.3 (100%) [M+2H]²⁺, 1286.7 (34%) [M+H]⁺.

### 1-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-2-{4-[(E)-(4-nitrophenyl)diazenyl]phenoxy}ethanone 6a.

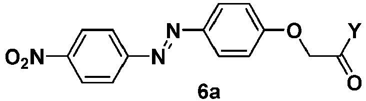

The synthesis of compound **6a** was carried out on 2.0 mmol scale of species **6** (0.60 g) using anhydrous tetrahydrofuran (30 mL), compound **1a** (1.15 g, 2.2 mmol) and **10** (0.61 g, 2.2 mmol) according to "Method G". Compound **6a** (1.7 g, 98.8%) was obtained as foamy orange solid, mp 86-88 °C; *δ*_{H} (300 MHz, CDCl₃): 8.37 (2H, d, *J* 9.0 Hz), 8.00 (2H, d, *J* 8.8 Hz), 7.98 (2H, d, *J* 9.0 Hz), 7.10 (2H, d, *J* 8.8 Hz), 4.82 (2H, s), 4.43-4.23 (4H, m), 4.20-3.99 (7H, m), 3.94-3.82 (2H, m), 3.77-3.52 (5H, m), 3.46 (3H, s), 3.45 (3H, s), 2.85-2.38 (5H, m), 1.51 (3H, s), 1.50 (3H, s), 1.40 (3H, s), 1.38 (3H, s), 1.35 (3H, s), 1.33 (3H, s); ESI (m/z, +c): 860.7 (100%) [M+H]⁺, 882.7 (60%) [M+Na]⁺.

### 1-[4-(6'-Deoxy)(lactose-6'-yl)piperazin-1-yl]-2-{4-[(E)-(4-nitrophenyl)diazenyl]-phenoxy}ethanone hydrochloride 6b.

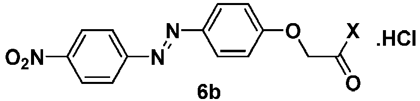

The synthesis of compound **6b** was carried out on 2.0 mmol scale of species **6a** (1.70 g) using acetone (30 mL) and hydrochloric acid (0.41 mL, 37% w/w, 5.0 mmol) dissolved in acetone (5 mL) according to "Method I". Compound **6b** (1.32 g, 90.4%) was obtained as orange solid, mp 187-215 °C (dec.); *λ*ₘₐₓ (ε) (H₂O): 365 nm (15100 M⁻¹cm⁻¹); *δ*_{H} (300 MHz, DMSO-d₆): 8.43 (2H, d, *J* 8.7 Hz), 8.04 (2H, d, *J* 8.7 Hz), 7.98 (2H, d, *J* 8.8 Hz), 7.20 (2H, d, *J* 8.8 Hz), 5.20-4.86 (4H, m), 4.58-4.26 (4H, m), 4.16-3.85 (7H, m), 3.83-2.90 (19H, m); ESI (m/z, +c): 694.5 (100%) [M+H]⁺, 716.5 (20%) [M+Na]⁺, 1409.2 (18%) [2M+Na]⁺.

### 1-Amino-4-{2-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-2-oxoethoxy}-2-phenoxyanthracene-9,10-dione 7a.

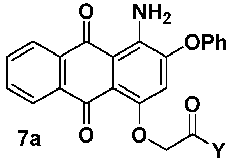

The synthesis of compound **7a** was carried out on 1.0 mmol scale of species **7** (0.39 g) using anhydrous tetrahydrofuran (15 mL), compound **1a** (0.58 g, 1.1 mmol) and **10** (0.30 g, 1.1 mmol) according to "Method G". Compound **7a** (0.83 g, 87.4%) was obtained as foamy red solid, mp 95-98 °C; *δ*_{H} (200 MHz, CDCl₃): 8.32-8.17 (2H, m), 7.80-7.71 (2H, m), 7.51-7.44 (2H, m), 7.31-7.28 (1H, m), 7.17-7.10 (2H, m), 6.77 [1H, s (br)], 4.66 [2H, s (br)], 4.44-4.25 (4H, m), 4.17-3.99 (5H, m), 3.91-3.78 (2H, m), 3.68-3.36 (7H, m), 3.43 [6H, s (br)], 2.77-2.39 (4H, m), 1.70 [1H, s (br)], 1.50 [6H, s (br)], 1.39 [3H, s (br)], 1.37 [3H, s (br)], 1.34 (3H, s), 1.32 (3H, s); ESI (m/z, +c): 948.9 (100%) [M+H]⁺, 970.5 (7%) [M+Na]⁺.

### 1-Amino-4-{2-[4-(6'-deoxy)(lactose-6'-yl)piperazin-1-yl]-2-oxoethoxy}-2-phenoxyanthracene-9,10-dione hydrochloride 7b.

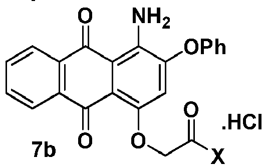

The synthesis of compound **7b** was carried out on 0.80 mmol scale of species **7a** (0.76 g) using acetone (10 mL) and hydrochloric acid (0.20 mL, 37% w/w, 2.40 mmol) dissolved in acetone (1.0 mL) according to "Method I". Compound **7b** (0.62 g, 95.4%) was obtained as red solid, mp 175-195 °C (dec.); *λ*ₘₐₓ (ε) (H₂O): 505 nm (6200 M⁻¹cm⁻¹); *δ*_{H} (300 MHz, DMSO-d₆): 8.27-8.09 (2H, m), 7.97-7.81 (2H, m), 7.61-7.46 (2H, m), 7.35-7.27 (1H, m), 7.24-7.15 (2H, m), 6.80 (1H, m), 4.97-4.71 (4H, m), 4.66-3.88 (6H, m), 3.81-3.66 (3H, m), 3.63-3.24 (15H, m), 3.17-2.72 (3H, m); ESI (m/z, +c): 782.6 (100%) [M+H]⁺, 804.5 (40%) [M+Na]⁺.

### (S)-2-(4-Amino-9,10-dioxo-3-phenoxy-9,10-dihydroanthracen-1-oxyl)-N-{1,5-bis-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-1,5-dioxopentan-2-yl}acetamide 8a.

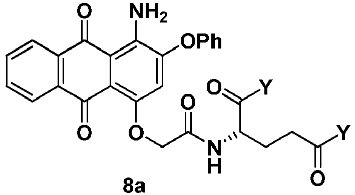

The synthesis of compound **8a** was carried out on 1.85 mmol scale of species **8** (0.96 g) using anhydrous tetrahydrofuran (30 mL), compound **1a** (2.35 g, 4.07 mmol) and **10** (1.13 g, 4.07 mmol) according to "Method G". Compound **8a** (2.26 g, 74.6%) was obtained as foamy pinkish-red solid, mp 104-106 °C; *δ*_{H} (200 MHz, CDCl₃): 8.39-8.19 (2H, m), 7.85-7.64 (2H, m), 7.55-7.41 (2H, m), 7.38-7.23 (2H, m), 7.18-7.08 (2H, m), 4.46-4.22 (9H, m), 4.17-3.92 (15H, m), 3.89-3.31 (26H, m), 2.78-2.30 (15H, m), 1.49 [12H, s(br)], 1.43 (3H, s), 1.38-1.30 (24H, m); ESI (m/z, +c): 819.0 (100%) [M+2H]²⁺, 1635.8 (25%) [M+H]⁺.

### (S)-2-(4-Amino-9,10-dioxo-3-phenoxy-9,10-dihydroanthracen-1-oxyl)-N-{1,5-bis-[4-(6'-deoxy)(lactose-6'-yl)piperazin-1-yl]-1,5-dioxopentan-2-yl}acetamide ditrifluoroacetate 8b.

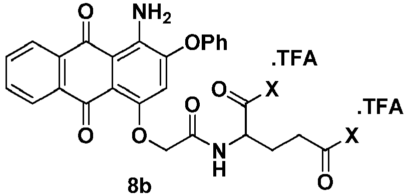

The synthesis of compound **8b** was carried out on 1.34 mmol scale of species **8a** (2.2 g) using 90% aq. (v/v) 2,2,2-trifluoroacetic acid (45 mL), toluene and acetone, according to "Method J". Compound **8b** (1.9 g, 92.7%) was obtained as red solid, mp 170-185 °C (dec.); *λ*ₘₐₓ (ε) (H₂O): 514 nm (4000 M⁻¹cm⁻¹); *δ*_{H} (200 MHz, DMSO-d₆): 8.36-8.07 (2H, m), 7.99-7.81 (2H, m), 7.65-7.16 (6H, m), 6.69-5.13 (18H, m), 5.08-4.18 (15H, m), 4.14-2.78 (30H, m), 2.63-2.53 (4H, m); ESI (m/z, +c): 652.4 (100%) [M+2H]²⁺, 1303.5 (65%) [M+H]⁺, 1325.4 (34%) [M+Na]⁺.

### 1-{3-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-3-oxopropylamino}anthracene-9,10-dione 9a.

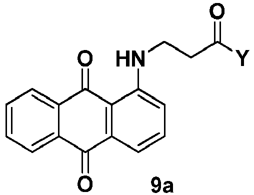

Compound **9a** was synthesized on 1.0 mmol scale of species **9** (0.30 g) using anhydrous tetrahydrofuran (15 mL), compound **1a** (0.63 g, 1.1 mmol) and compound **10** (0.30 g, 1.1 mmol) according to "Method G". Compound **9a** (0.81 g, 95.0%) was obtained as foamy red solid, mp 72-74.5 °C; *δ*_{H} (200 MHz, CDCl₃): 9.91-9.78 (1H, m), 8.31-8.19 (2H, m), 7.82-7.67 (2H, m), 7.66-7.52 (2H, m), 7.18-7.08 (1H, m), 4.45-4.23 (3H, m), 4.19-3.97 (6H, m), 3.93-3.80 (3H, m), 3.77-3.62 (4H, m), 3.60-3.37 (10H, m), 2.87-2.39 (6H, m), 1.68 (1H, br), 1.50 [6H, s (br)], 1.39 [3H, s (br)], 1.37 [3H, s (br)], 1.34 (3H, s), 1.32 (3H, s); ESI (m/z, +c): 854.9 (100%) [M+H]⁺.

### 1-{3-[4-(6'-Deoxy)(lactose-6'-yl)piperazin-1-yl]-3-oxopropylamino}anthracene-9,10-dione hydrochloride 9b.

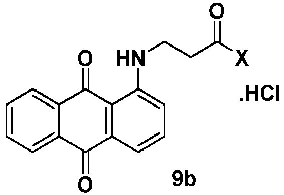

The synthesis of compound **9b** was carried out on 0.94 mmol scale of species **9a** (0.80 g) using acetone (10 mL) and hydrochloric acid (0.23 mL, 37% w/w, 2.82 mmol) dissolved in acetone (1.00 mL) according to "Method I". Compound **9b** (0.67 g, 98.5%) was obtained as red solid, mp 130-155 °C (dec.); *λ*ₘₐₓ (ε) (H₂O): 514 nm (3000 M⁻¹cm⁻¹); *δ*_{H} (300 MHz, DMSO-d₆): 9.83 [1H, s (br)], 8.26-8.11 (2H, m), 7.98-7.81 (2H, m), 7.75-7.64 (1H, m), 7.51-7.44 (1H, m), 7.35-7.31 (1H, m), 4.95-4.16 [6H, m (br)], 4.15-3.88 (6H, m), 3.80-3.44 (13H, m), 3.40-3.03 (12H, m), 3.00-2.68 (4H, m); ESI (m/z, +c): 688.6 (100%) [M+H]⁺, 610.5 (27%) [M+H]⁺.

### Diethyl 4,4'-(m-tolylazanediyl)dibutanoate 11.

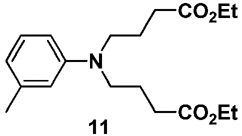

m-Chloroaniline (1.61 g, 15 mmol) was dissolved in dimethylformamide (7.5 mL) and potassium carbonate (7.05 g, 51 mmol) was added at 20 °C, followed by ethyl 4-bromobutyrate (8.78 g, 45.0 mmol). The resulting mixture was warmed to 100 °C for 6 h, after which it was cooled to 20 °C. The slurry was partitioned between water and diethylether and the organic phase was separated, further washed with water and brine, dried over anhydrous sodium sulphate and filtered. The filtrate was evaporated *in vacuo* and the crude oil was purified by flash column chromatography [gradient 5-10% ethyl acetate / petrol ether (bp: 40-60 °C)] isolating the title compound (4.70 g, 93.4%) as pale yellow oil; R_{f} [10% ethyl acetate / petrol ether (bp: 40-60 °C)] 0.32; *δ*_{H} (200 MHz, CDCl₃): 7.16-7.07 (1H, m), 6.58-6.47 (3H, m), 4.15 (4H, q, *J* 7.0 Hz), 3.36-3.29 (4H, m), 2.39-2.32 (7H, m), 2.02-1.84 (4H, m), 1.27 (6H, t, *J* 7.0 Hz); ESI (m/z, +c): 336.1 (100%) [M+H]⁺.

### Diethyl 4,4'-{4-[(E)-(2-cyano-4-nitrophenyl)diazenyl]-3-methylphenylazanediyl}dibutanoate 11a.

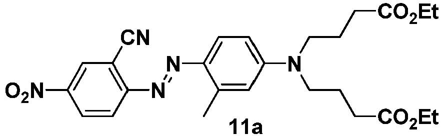

The synthesis of compound **11a** was carried out on 2.0 mmol scale of 2-amino-5-nitrobenzonitrile (0.33 g) using acetic acid (4.3 mL), propionic acid (0.8 mL), nitrous sulfuric anhydride (2 mmol, 0.64 g, 40% w/w in conc. sulphuric acid), compound **11** (0.71 g, 2 mmol), ethanol (10 mL) and ice (10 g) according to "Method C". Compound **11a** (0.84 g, 82.4%) was obtained as dark purple solid, mp 87-89.5 °C; *δ*_{H} (200 MHz, CDCl₃): 8.61 (1H, d, *J* 2.4 Hz), 8.42 (1H, dd, *J* 9.2 and *J* 2.4 Hz), 8.06 [1H, d (br), *J* 9.2 Hz], 7.98 (1H, d, *J* 9.2 Hz), 6.77-6.64 (2H, m), 4.20 (4H, q, *J* 7.0 Hz), 3.55-3.47 (4H, m), 2.73 (3H, s), 2.41 (4H, t, *J* 7.0 Hz), 2.08-1.88 (4H, m), 1.29 (6H, t, *J* 7.0 Hz); ESI (m/z, +c): 510.5 (10%) [M+H]⁺, 532.5 (7%) [M+Na]⁺, 1040.7 (100%) [2M+Na]⁺.

### 4,4'-{4-[(E)-(2-Cyano-4-nitrophenyl)diazenyl]-3-methylphenylazanediyl}dibutanoic acid 11b.

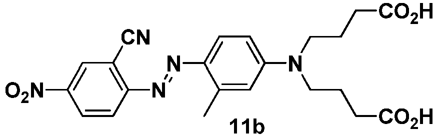

The synthesis of compound **11b** was carried out on 1.49 mmol scale of compound **11a** (0.76 g) using tetrahydrofuran (15 mL), an aq. 1 M solution of potassium hydroxide (0.34 g, 6.0 mmol) and 1 M hydrochloric acid according to "Method D". Compound **11b** (0.66 g, 97.0%) was obtained as dark purple solid, mp 219-222 °C; *δ*_{H} (300 MHz, DMSO-d₆): 8.79 (1H, d, *J* 2.4 Hz), 8.51 (1H, dd, *J* 9.3 and *J* 2.4 Hz), 7.95 (1H, d, *J* 9.3 Hz), 7.84-7.80 (1H, m), 6.90-6.88 (2H, m), 3.52-3.47 (4H, m), 2.66 (3H, s), 2.34 (4H, t, *J* 7.2 Hz), 1.92-1.73 (4H, m); ESI (m/z, -c): 452.5 (22%) [M-1]⁻, 905.3 (100%) [2M-1]⁻.

### 2-{(E)-{4-{Bis-{4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutyl}amino}-2-methylphenyl}diazenyl}-5-nitrobenzonitrile 11c.

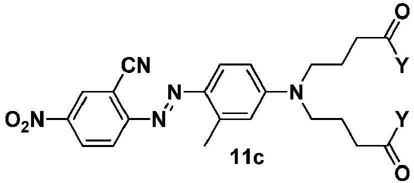

The synthesis of compound **11c** was carried out on 1.32 mmol scale of species **11b** (0.60 g) using anhydrous tetrahydrofuran (15 mL), compound **1a** (1.67 g, 2.90 mmol) and **10** (0.80 g, 2.90 mmol) according to "Method G". Compound **11c** (2.0 g, 98.0%) was obtained as foamy dark purple solid, mp 105-107 °C; *δ*_{H} (300 MHz, CDCl₃): 8.44 (1H, d, *J* 2.4 Hz), 8.25 (1H, dd, *J* 9.0 and *J* 2.4 Hz), 7.86-7.83 (1H, m), 7.78 (1H, d, *J* 9.0 Hz), 6.63-6.48 (2H, m), 4.28-4.06 (6H, m), 4.05-3.60 (19H, m), 3.58-3.1 (26H, m), 2.65-2.13 (18H, m), 1.07-1.77 (4H, m), 1.34 [12H, s (br)], 1.24 (6H, s), 1.22 (6H, s), 1.18 (6H, s), 1.17 (6H, s); ESI (m/z, +c): 797.4 (82%) [M+H+Na]²⁺, 808.7 (86%) [M+2Na]²⁺, 1570.9 (39%) [M+H]⁺, 1593.3 (100%) [M+Na]⁺.

### 2-{(E)-{4-{Bis-{4-[4-(6'-deoxy)(lactose-6'-yl)piperazin-1-yl]-4-oxobutyl}amino}-2-methylphenyl}diazenyl}-5-nitrobenzonitrile ditrifluoroacetate 11d.

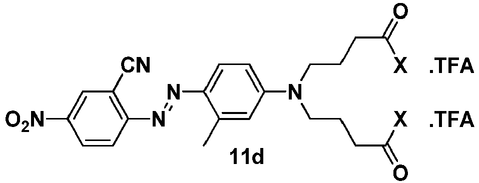

The synthesis of compound **11d** was carried out on 1.23 mmol scale of species **11c** (1.90 g) using 90% aq. (v/v) 2,2,2-trifluoroacetic acid (40 mL), toluene and acetone, according to "Method J". Compound **11d** (1.59 g, 89.8%) was obtained as dark purple solid, mp 100-124 °C (dec.); *λ*ₘₐₓ (ε) (H₂O): 553 nm (25400 M⁻¹cm⁻¹); *δ*_{H} (300 MHz, DMSO-d₆): 8.81 (1H, d, *J* 2.4 Hz), 8.53 (1H, dd, *J* 9.0 and *J* 2.4 Hz), 7.96 (1H, d, *J* 9.0 Hz), 7.86 (1H, d, *J* 9.0 Hz), 7.08-6.90 (2H, m), 5.63-4.20 (28H, m), 4.17-2.78 (53H, m), 2.76-2.36 (18H, m), 1.95-1.70 (4H, m); ESI (m/z, +c): 620.4 (100%) [M+2H]²⁺, 1238.7 (23%) [M+H]⁺.

### (E)-2-{[2-Cyanoethyl]-{4-[(2,6-dichloro-4-nitrophenyl)diazenyl]phenyl}amino}-ethyl acetate 12.

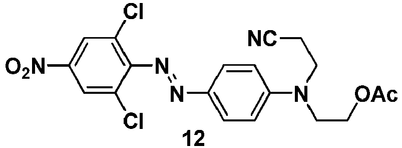

Commercial Disperse Orange 30 (Foron Yellow Brown S-2RFL 150) (100 g) was purified using dichloromethane (1.0 L) according to "Method A". Compound **12** (45.5 g, 45.5%) was obtained as dark orange solid, mp 123-125.5 °C; *δ*_{H} (200 MHz, CDCl₃): 8.28 (2H, s), 7.97 (2H, d, *J* 9.2 Hz), 6.85 (2H, d, *J* 9.2 Hz), 4.33 (2H, t, *J* 6.0 Hz), 3.94-3.79 (4H, m), 2.74 (2H, t, *J* 6.8 Hz), 2.08 (3H, s); ESI (m/z, +c): 450.1 (59%) [M+H]⁺, 452.2 (40%) [M+H]⁺, 472.2 (100%) [M+Na]⁺, 474.1 (46%) [M+Na]⁺.

### (E)-3-{{4-[(2,6-Dichloro-4-nitrophenyl)diazenyl]phenyl}-(2-hydroxyethyl)amino}propanenitrile 12a.

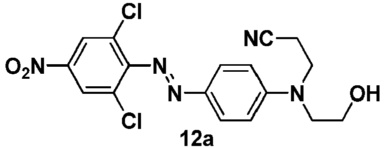

Compound **12** (10.0 g, 22.2 mmol) was dissolved in methanol (80 mL) and an aq. solution (40 mL) of potassium carbonate (3,68 g, 26.6 mmol) was added portionwise at 0 °C. The resulting mixture was stirred for 0.3 h, it was warmed to 20 °C and stirred overnight. The solvent was evaporated under reduced pressure and the crude residue was slurried in methanol and water (150 mL, 1:5 v/v). The fine suspension was filtered under suction and the press cake was washed with water and dried in air, to obtain compound **12a** (8.63 g, 95.0%) as dark orange solid, mp 125-127 °C; *δ*_{H} (300 MHz, CDCl₃): 8.28 (2H, s), 7.96 (2H, d, *J* 9.3 Hz), 6.83 (2H, d, *J* 9.3 Hz), 4.01-3.88 (4H, m), 3.75 (2H, t, *J* 5.4 Hz), 2.79 (2H, t, *J* 6.9 Hz); ESI (m/z, +c): 408.2 (100%) [M+H]⁺, 410.1 (51%) [M+H]⁺.

### (E)-4-{2-[(2-Cyanoethyl)-{4-[(2,6-dichloro-4-nitrophenyl)diazenyl]phenyl}amino]ethoxy}-4-oxobutanoic acid 12b.

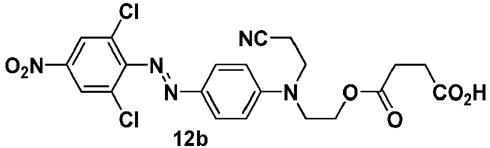

The synthesis of compound **12b** was carried out on 35.0 mmol scale of species **12a** (14.3 g) using tetrahydrofuran (170 mL), dihydrofuran-2,5-dione (4.2 g, 42.0 mmol), *N,N*-dimethylpyridin-4-amine (0.43 g, 3.52 mmol), triethylamine (4.6 g, 45.5 mmol) and acetic acid (10.5 g, 175 mmol) according to "Method E". Compound **12b** (17.9 g, 96.3%) was obtained as dark red solid, mp 125-127 °C; *δ*_{H} (300 MHz, CDCl₃): 8.26 (2H, s), 7.95 (2H, d, *J* 9.3 Hz), 6.82 (2H, d, *J* 9.3 Hz), 4.38 (2H, t, *J* 5.7Hz), 3.88-3.80 (4H, m), 2.72 (2H, t, *J* 6.9 Hz), 2.71-2.59 (4H, m); ESI (m/z, -c): 506.0 (14%) [M-H]⁻, 508.0 (7%) [M-H]⁻, 1013.0 (77%) [2M-H]⁻, 1014.9 (100%) [2M-H]⁻, 1017.1 (58%) [2M-H]⁻, 1019.1 (9%) [2M-H]⁻.

### 2-{[2-Cyanoethyl]-{4-[(E)-(2,6-dichloro-4-nitrophenyl)diazenyl]phenyl}amino}-ethyl 4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanoate 12c.

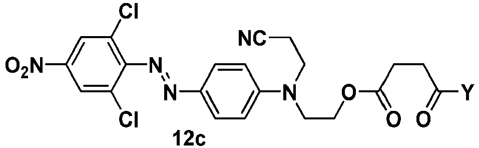

The synthesis of compound **12c** was carried out on 8.0 mmol scale of species **12b** (4.07 g) using anhydrous tetrahydrofuran (100 mL), compound **1a** (5.3 g, 9.2 mmol) and compound **10** (2.40 g, 8.7 mmol) according to "Method G". Compound **12c** (8.20 g, 96.1%) was obtained as foamy orange solid, mp 133-134.5 °C; *δ*_{H} (300 MHz, CDCl₃): 8.28 (2H, s), 7.96 (2H, d, *J* 9.3 Hz), 6.83 (2H, d, *J* 9.3 Hz), 4.40-4.25 (6H, m), 4.18-4.00 (5H, m), 3.96-3.82 (7H, m), 3.62-3.52 (3H, m), 3.48-3.41 (2H, m), 3.46 (3H, s), 3.45 (3H, s), 2.78-2.73 (4H, m), 2.64-2.43 (8H, m), 1.51 (3H, s), 1.50 (3H, s), 1.40 (3H, s), 1.38 (3H, s), 1.35 (3H, s), 1.33 (3H, s); ESI (m/z, +c): 1066.8 (100%) [M+H]⁺, 1088.6 (10%) [M+Na]⁺.

### 2-{[2-Cyanoethyl]-{4-[(E)-(2,6-dichloro-4-nitrophenyl)diazenyl]phenyl}amino}-ethyl 4-[4-(6'-deoxy)(lactose-6'-yl)piperazin-1-yl]-4-oxobutanoate hydrochloride 12d.

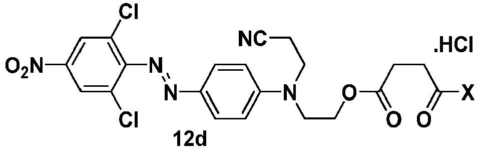

The synthesis of compound **12d** was carried out on 8.0 mmol scale of species **12c** (8.5 g) using acetone (100 mL) and hydrochloric acid (1.66 mL, 37% w/w, 20.0 mmol) dissolved in acetone (15 mL) according to "Method I". Compound **12d** (6.78 g, 90.4%) was obtained as dark orange solid, mp 174-183 °C (dec.); *λ*ₘₐₓ (ε) (H₂O): 441 nm (16300 M⁻¹cm⁻¹); *δ*_{H} (300 MHz, DMSO-d₆): 8.44 (2H, s), 7.85 (2H, d, *J* 9.3 Hz), 7.06 (2H, d, *J* 9.3 Hz), 5.13-4.52 (8H, m), 4.43-4.21 (4H, m), 4.14-3.95 (2H, m), 3.93-3.40 (13H, m), 3.37-2.92 (11H, m), 2.90-2.82 (2H, s), 2.70-2.60 (1H, m), 2.58-2.53 (1H, s); ESI (m/z, +c): 900.2 (100%) [M+H]⁺, 902.3 (89%) [M+H]⁺, 904.0 (43%) [M+H]⁺, 922.3 (18%) [M+Na]⁺, 924.2 (12%) [M+Na]⁺.

### (E)-2-{Ethyl-{4-[(4-fluorophenyl)diazenyl]phenyl}amino}ethanol 13.

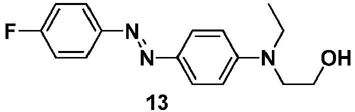

An ice-cooled solution of sodium nitrite (3.45 g, 50.0 mmol, 30% aq. w/w) was added to a solution of 4-fluoroaniline (5.55 g, 50.0 mmol) dissolved in 6 M hydrochloric acid (30 mL) at 0 °C. The mixture was stirred for 0.5 h and a solution of 2-[ethyl(phenyl)amino]ethanol (8.26 g, 50.0 mmol) dissolved in 6 M hydrochloric acid (30 mL) was added dropwise. The resulting deep coloured slurry was warmed to 20 °C over 1 h: then, it was treated with 3 M sodium acetate (50 mL). The fine slurry was filtered under suction and the press cake was washed thoroughly with water and dried in air. Compound **13** (13 g, 90.5%) was obtained as bright orange solid, mp 106-107 °C; *δ*_{H} (200 MHz, CDCl₃): 7.90-7.79 (4H, m), 7.22-7.10 (2H, m), 6.85-6.75 (2H, m), 3.87 (2H, t, *J* 5.8 Hz), 3.64-3.46 (4H, m), 1.24 (3H, t, *J* 7.0 Hz); ESI (m/z, +c): 288.2 (100%) [M+H]⁺.

### (E)-4-{2-{Ethyl-{4-[(4-fluorophenyl)diazenyl]phenyl}amino}ethoxy}-4-oxobutanoic acid 13a.

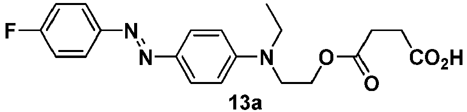

The synthesis of compound **13a** was carried out on 45.2 mmol scale of species **13** (13 g) using dichloromethane (190 mL), dihydrofuran-2,5-dione (5.0 g, 49.7 mmol), *N,N*-dimethylpyridin-4-amine (0.55 g, 4.5 mmol), triethylamine (5.03 g, 49.7 mmol) and acetic acid (13.5 g, 225 mmol) according to "Method E". Compound **13a** (17 g, 97.1%) was obtained as bright yellow solid, mp 108.5-110 °C; *δ*_{H} (200 MHz, CDCl₃): 7.90-7.80 (4H, m), 7.22-7.10 (2H, m), 6.78 (2H, d, *J* 9.2 Hz), 4.32 (2H, t, *J* 6.4 Hz), 3.66 (2H, t, *J* 6.2 Hz), 3.52 (2H, q, *J* 7.0 Hz), 2.75-2.58 (4H, m), 1.24 (3H, t, *J* 7.0 Hz); ESI (m/z, -c): 773.2 (100%) [2M-H]⁻.

### 2-{Ethyl-{4-[(E)-(4-fluorophenyl)diazenyl]phenyl}amino}ethyl 4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanoate 13b.

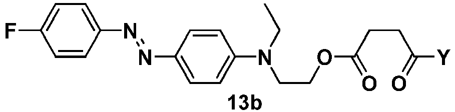

The synthesis of compound **13b** was carried out on 7.74 mmol scale of species **13a** (3.0 g) using anhydrous dichloromethane (30 mL), sulfurous dichloride (1.01 g, 8.50 mmol) and compound **1a** (4.46 g, 7.74 mmol) dissolved in anhydrous dichloromethane (30 mL) containing triethylamine (2.35 g, 23.2 mmol) according to "Method F". Compound **13b** (6.9 g, 94.2%) was obtained as foamy orange solid, mp 104-105 °C; *δ*_{H} (200 MHz, CDCl₃): 7.87-7.81 (4H, m), 7.19-7.11 (2H, m), 6.78 (2H, d, *J* 9.0 Hz), 4.41-4.25 (6H, m), 4.17-4.00 (5H, m), 3.93-3.82 (2H, m), 3.69 (2H, t, *J* 6.8 Hz), 3.72-3.42 (9H, m), 3.46 (3H, s), 3.45 (3H, s), 2.80-2.40 (11H, m), 1.51 (3H, s), 1.50 (3H, m), 1.39 (3H, m), 1.38 (3H, m), 1.35 (3H, m), 1.33 (3H, m), 1.24 (3H, t, *J* 6.9 Hz); ESI (m/z, +c): 946.7 (96%) [M+H]⁺, 968.7 (100%) [M+Na]⁺.

### 2-{Ethyl-{4-[(E)-(4-fluorophenyl)diazenyl]phenyl}amino}ethyl 4-[4-(6'-deoxy)(lactose-6'-yl)piperazin-1-yl]-4-oxobutanoate hydrochloride 13c.

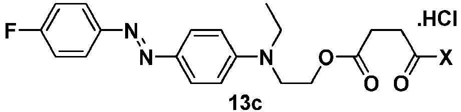

The synthesis of compound **13c** was carried out on 7.29 mmol scale of species **13b** (6.9 g) using acetone (75 mL) and hydrochloric acid (1.51 mL, 37% w/w, 18.2 mmol) dissolved in acetone (15 mL) according to "Method I". Compound **13c** (5.1 g, 81.1%) was obtained as dark yellow-orange solid, mp 128-140 °C (dec.); *λ*ₘₐₓ (ε) (H₂O): 452 nm (39200 M⁻¹cm⁻¹); *δ*_{H} (300 MHz, DMSO-d₆): 7.88-7.74 (4H, m), 7.41-7.31 (2H, m), 6.89 (2H, d, *J* 9.0 Hz), 4.92-4.28 (10H, m), 4.26-4.18 (3H, m), 4.16-3.82 (4H, m), 3.80-3.20 (17H, m), 3.19-2.80 (3H, m), 2.79-2.37 (3H, m), 1.15 (3H, t, *J* 6.9 Hz); ESI (m/z, +c): 780.4 (100%) [M+H]⁺, 802.5 (60%) [M+Na]⁺.

### (E)-4-{[4-(Dimethylamino)phenyl]diazenyl}benzoic acid 14.

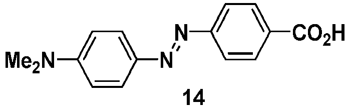

A solution of sodium nitrite (0.69 g, 10.0 mmol, 20% aq. w/w in water) was added to a solution of 4-aminobenzoic acid (1.37 g, 10.0 mmol) dissolved in 1.5 M hydrochloric acid (25 mL) at 0 °C. The mixture was stirred for 0.5 h and a solution of *N',N'*-dimethylbenzene-1,4-diamine (1.21 g, 10.0 mmol) dissolved in 1.8 M hydrochloric acid (10 mL) was added dropwise. The resulting deep coloured slurry was stirred for 0.5 h and an aq. 8 M solution of sodium hydroxide was added to raise the pH to 3-4. The slurry was warmed to 20 °C over 1 h, diluted with water (40 mL) and filtered. The press cake was washed thoroughly with water and dried in air, to isolate compound **14** (2.47 g, 91.8%) as bright red-orange solid, mp 252-254 °C; *δ*_{H} (200 MHz, DMSO-d₆): 8.05 (2H, d, *J* 8.4 Hz), 7.80 (4H, m), 6.83 (2H, d, *J* 9.2 Hz), 3.60 [1H, s (br)], 3.06 (6H, s); ESI (m/z, +c): 268.7 (100%) [M+1]⁺.

### {4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-{4-{(E)-[4-(dimethylamino)phenyl]diazenyl}phenyl}methanone 14a.

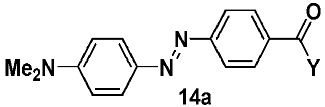

The synthesis of compound **14a** was carried out on 2.0 mmol scale of species **14** (0.54 g) using anhydrous tetrahydrofuran (30 mL), compound **1a** (1.15 g, 2.2 mmol) and **10** (0.61 g, 2.2 mmol) according to "Method G". Compound **14a** (1.6 g, 97.0%) was obtained as foamy orange-red solid, mp 96-99 °C; *δ*_{H} (300 MHz, CDCl₃): 7.88 (2H, d, *J* 9.2 Hz), 7.85 (2H, d, *J* 8.4 Hz), 7.51 (2H, d, *J* 8.4 Hz), 6.76 (2H, d, *J* 9.2 Hz), 4.46-4.33 (2H, m), 4.32-4.23 (2H, m), 4.21-4.00 (5H, m), 3.97-3.80 (4H, m), 3.62-3.50 (2H, m), 3.49-3.37 (2H, m), 3.43 (3H, s), 3.41 (3H, s), 3.11 (6H, s), 2.80-2.78 (2H, m), 2.70-2.39 (4H, m), 1.51 (3H, s), 1.49 (3H, s), 1.38 (3H, s), 1.37 (3H, s), 1.34 (3H, s), 1.33 (3H, s); ESI (m/z, +c): 828.6 (100%) [M+H]⁺, 1654.5 (21%), [2M+H]⁺.

### [4-(6'-Deoxy)(lactose-6'-yl)piperazin-1-yl}-{4-{(E)-[4-(dimethylamino)phenyl]-diazenyl}phenyl}methanone hydrochloride 14b.

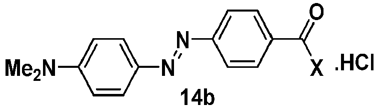

The synthesis of compound **14b** was carried out on 2.0 mmol scale of species **14a** (1.66 g) using acetone (25 mL) and hydrochloric acid (0.50 mL, 37% w/w, 6.0 mmol) dissolved in acetone (5 mL) according to "Method I". Compound **14b** (1.28 g, 91.4%) was obtained as red solid, mp 350-370 °C (dec.); *λ*ₘₐₓ (ε) (H₂O): 464 nm (16700 M⁻¹cm⁻¹); *δ*_{H} (300 MHz, DMSO-d₆): 7.85 (2H, d, *J* 8.4 Hz), 7.84 (2H, d, *J* 9.0 Hz), 7.62 (2H, d, *J* 8.4 Hz), 6.88 (2H, d, *J* 9.0 Hz), 4.97-4.14 [10H, m (br)], 4.13-3.84 (3H, m), 3.81-3.45 (8H, m), 3.42-3.14 (10H, m), 3.09 (6H, s); ESI (m/z, +c): 331.8 (12%) [M+2H]²⁺, 662.4 (100%) [M+H]⁺, 1323.4 (6%) [2M+H]⁺.

### N-{4-{(E)-{4-[Bis-(2-hydroxyethyl)amino]phenyl}diazenyl}phenyl}-4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanamide 15.

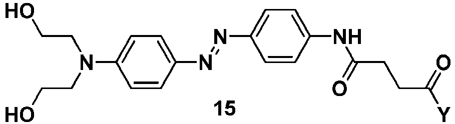

In a 25 ml one neck round bottomed flask Disperse Black 9 (0.29 g, 0.95 mmol) was dissolved in anhydrous tetrahydrofuran (10 mL) and compound **1b** (0.64 g, 0.95 mmol) was added, followed by compound **10** (0.29 g, 1.05 mmol). The mixture was stirred at 20 °C for 72 h, after which the solvent was evaporated *in vacuo* and the crude residue was partitioned between ethyl acetate and water. The organic phase was separated, washed with brine, dried over anhydrous sodium sulfate and filtered, to obtain compound **15** (0.82 g, 90.0%) as foamy orange solid, mp 83-85 °C; *δ*_{H} (200 MHz, CDCl₃): 10.2 [1H, s (br)], 7.90-7.22 (6H, m), 6.85-6.68 (2H, m), 4.48-4.23 (3H, m), 4.18-4.02 (5H, m), 3.99-3.81 (9H, m), 3.79-3.60 (8H, m), 3.58-3.37 (11H, m), 2.86-2.39 (8H, m), 1.50 [6H, s (br)], 1.38 [6H, s (br)], 1.33 [6H, s (br)]; ESI (m/z, +c): 480.7 (26%) [M+2H]²⁺, 959.6 (100%) [M+H]⁺, 981.7.

### N-{4-{(E)-{4-[Bis-(2-hydroxyethyl)amino]phenyl}diazenyl}phenyl}-4-[4-(6'-deoxy)(lactose-6'-yl)piperazin-1-yl]-4-oxobutan-amide hydrochloride 15a.

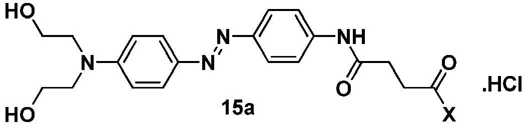

The synthesis of compound **15a** was carried out on 0.72 mmol scale of species **15** (0.69 g) using acetone (10 mL) and hydrochloric acid (0.15 mL, 37% w/w, 1.8 mmol) dissolved in acetone (1.0 mL) according to "Method I". Compound **15a** (0.55 g, 92.3%) was obtained as yellow-orange solid, mp 260-285 °C (dec.); *λ*ₘₐₓ (ε) (H₂O): 459 nm (20700 M⁻¹cm⁻¹); *δ*_{H} (300 MHz, DMSO-d₆): 10.3 [1H, s (br)], 7.97-7.63 (6H, m), 6.98-6.78 (2H, m), 5.58-4.56 [11H, m (br)], 4.48-3.82 [6H, m (br)], 3.78-3.41 [14H, m (br)], 3.41-3.33 [8H, m (br)], 2.86-2.38 [4H, m (br)]; ESI (m/z, +c): 397.5 (100%) [M+2H]²⁺, 793.5 (79%) [M+H]⁺, 815.6 (44%) [M+Na]⁺.

### 2-Nitro-4-(pyrrolidin-1-yl)aniline 16.

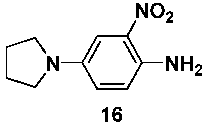

4-Fluoro-3-nitroaniline (0.47 g, 3 mmol) was dissolved in acetonitrile (10 mL) and potassium carbonate (0.58 g, 4.2 mmol) was added, followed by pyrrolidine (0.26 g, 3.6 mmol). The resulting slurry was heated to reflux for 3 h and then it was cooled to 20 °C. The mixture was diluted with ethyl acetate, filtered and evaporated under reduced pressure. The crude residue was partitioned between ethyl acetate and water and the organic phase was separated, washed with water, brine, filtered and eventually evaporated *in vacuo,* to obtain compound **16** (0.60 g, 96.5%) as bright orange solid; *δ*_{H} (200 MHz, CDCl₃): 7.20 (1H, d, *J* 2.6 Hz), 6.94-6.80 (2H, m), 3.74 [2H, s (br)], 3.24-3.05 (4H, m), 2.07-1.85 (4H, m).

### 4-[2-Nitro-4-(pyrrolidin-1-yl)phenylamino]-4-oxobutanoic acid 16a.

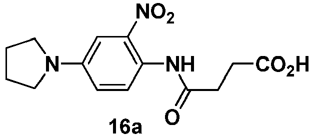

Compound **16** (0.59 g, 2.84 mmol) was dissolved in dichloromethane (10 mL) and dihydrofuran-2,5-dione (0.34 g, 3.41 mmol) was added followed by triethylamine (0.37 g, 3.69 mmol). The mixture was stirred at 20 °C for 6 h and acetic acid (1.1 g, 18.5 mmol) was added. The solvent was evaporated under reduced pressure and the residue was partitioned between ethyl acetate and water. The organic phase was separated, washed with brine, filtered and evaporated to dryness. The thick orange oil was slurried in petrol ether (bp: 40-60 °C) and the fine suspension was filtered under suction to obtain compound **16a** (0.85 g, 97.4%) as yellow-orange powder, mp 135-138 °C; *δ*_{H} (200 MHz, DMSO-d₆): 9.94 (1H, s), 8.14 (1H, d, *J* 2.6 Hz), 7.52 (1H, dd, *J* 9.0 and *J*' 2.6 Hz), 6.94 (1H, d, *J* 9.0 Hz), 3.17-3.00 [4H, m (br)], 2.53 [4H, s (br)], 1.99-1.81 [4H, m (br)]; ESI (m/z, +c): 308.2 (100%) [M+H]⁺.

### 4-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-N-[2-nitro-4-(pyrrolidin-1-yl)phenyl]-4-oxobutanamide 16b.

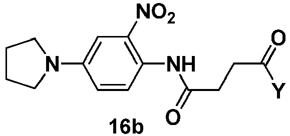

The synthesis of compound **16b** was carried out on 1.0 mmol scale of compound **16a** (0.31 g) using anhydrous tetrahydrofuran (10 mL), compound **1a** (0.63 g, 1.1 mmol) and **10** (0.30 g, 1.1 mmol) according to "Method G". Compound **16b** (0.79 g, 91.3%) was obtained as foamy orange solid, mp 99-101 °C; *δ*_{H} (200 MHz, CDCl₃): 8.76 [1H, s (br)], 7.93 (1H, d, *J* 2.6 Hz), 7.52 (1H, dd, *J* 9.2 and *J*' 2.6 Hz), 6.82 (1H, d, *J* 9.2 Hz), 4.45-4.24 (4H, m), 4.19-4.02 (5H, m), 3.93-3.82 (3H, m), 3.70-3.39 (6H, m), 3.46 (6H, s), 3.24-3.09 [4H, m (br)], 2.85-2.40 [8H, (m)], 1.90-1.81 (4H, m), 1.79 [1H, s (br)], 1.50 (6H, s), 1.39 (3H, s), 1.37 (3H, s), 1.34 (3H, s), 1.33 (3H, s); ESI (m/z, +c): 866.3 (100%) [M+H]⁺, 888.5 (40%) [M+Na]⁺.

### 4-[4-(6'-Deoxy)(lactose-6'-yl)piperazin-1-yl]-N-[2-nitro-4-(pyrrolidin-1-yl)phenyl]-4-oxobutanamide hydrochloride 16c.

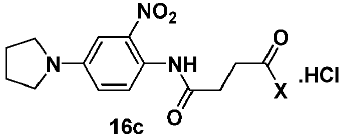

The synthesis of compound **16c** was carried out on 0.81 mmol scale of species **16b** (0.70 g) using acetone (8 mL) and hydrochloric acid (0.15 mL, 37% w/w, 1.75 mmol) dissolved in acetone (1 mL) according to "Method I". Compound **16b** (0.52 g, 87.2%) was obtained as pale orange powder, mp 180-220 °C (dec.); *λ*ₘₐₓ (ε) (H₂O): 461 nm (2300 M⁻¹cm⁻¹); *δ*_{H} (200 MHz, DMSO-d₆): 10.11 [1H, s (br)], 8.20 (1H, d, *J* 2.6 Hz), 7.55 (1H, dd, *J* 9.2 and *J*' 2.6 Hz), 7.03 (1H, d, *J* 9.2 Hz), 4.83-4.25 [10H, m (br)], 4.17-3.81 (4H, m), 3.80-3.21 [14H, m (br)], 3.17-3.00 [6H, m (br)], 2.78-2.56 (3H, m), 2.00-1.79 (4H, m); ESI (m/z, +c): 700.6 (100%) [M+H]⁺.

### 3-Nitro-N-phenyl-4-(phenylamino)benzenesulfonamide 17.

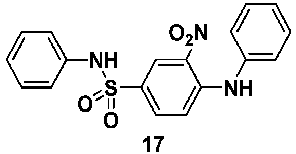

Commercial Disperse Yellow 42 (Teratop Yellow GWL, Huntsman) (100 g) was purified using dichloromethane (1.0 L) according to "Method A". Compound **17** (56.0 g, 56.0%) was obtained as fine bright yellow powder, mp 162-163 °C; *δ*_{H} (200 MHz, CDCl₃): 9.80 [1H, s (br)], 8.69 (1H, d, *J* 2.2 Hz), 7.63 (1H, dd, *J* 9.2 and *J'* 2.2 Hz), 7.52-7.42 (2H, m), 7.38-7.21 (4H, m), 7.19-7.07 (4H, m), 6.83 [1H, s (br)]; ESI (m/z, -c): 368.2 (100%) [M-1]⁻, 759.1 (12%) [2(M-H)+Na]⁻.

### Ethyl 2-{[2-nitro-4-(N-phenylsulfamoyl)phenyl](phenyl)amino} acetate 17a.

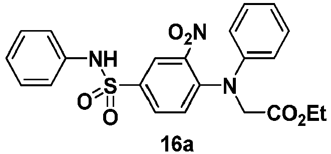

Compound **17** (10.0 g, 27.1 mmol) was dissolved in acetone (100 mL) and potassium carbonate (5.1 g, 36.5 mmol) was added followed by potassium iodide (0.23 g, 1.35 mmol). The suspension was warmed to 50 °C, ethyl 2-chloroacetate (3.98 g, 32.5 mmol) was added dropwise and the whole was refluxed for 3 h. The slurry was cooled to 20 °C, filtered under suction and the filtrate was evaporated to dryness under reduced pressure. The residue was partitioned between ethyl acetate and water: the organic phase was then separated, washed with brine and dried over anhydrous sodium sulphate. The suspension was filtered under suction and the filtrate was evaporated *in vacuo,* to yield the title compound (11.9 g, 96.7%) as bright yellow-orange powder, mp 108-110.5 °C; *δ*_{H} (300 MHz, CDCl₃): 9.82 [1H, s (br)], 8.59 (1H, d, *J* 2.1 Hz), 7.59 (1H, ddd, *J* 0.6 *J'* 2.4 and *J"* 9.3 Hz), 7.51-7.44 (2H, m), 7.37-7.28 (8H, m), 7.13 (1H, d, *J* 9.0 Hz), 4.46 (2H, s), 4.18 (2H, q, *J* 7.2 Hz), 1.25 (3H, t, *J* 7.2 Hz); ESI (m/z, +c): 456.2 (4%) [M+H]⁺, 478.3 (6%) [M+Na]⁺, 933.2 (100%) [2M+Na]⁺.

### 2-{[2-Nitro-4-(N-phenylsulfamoyl)phenyl](phenyl)amino}acetic acid 17b.

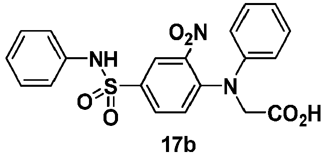

The synthesis of compound **17b** was carried out on 30.3 mmol scale of compound **17a** (13.8 g) using tetrahydrofuran (90 mL) an aq. 1 M solution of potassium hydroxide (3.40 g, 60.6 mmol) and 1 M hydrochloric acid according to "Method D". Compound **17b** (11.5 g, 89.0%) was obtained as bright yellow solid, mp 169-171 °C; *δ*_{H} (300 MHz, CDCl₃): 9.82 [1H, s (br)], 8.61 (1H, d, *J* 2.4 Hz), 7.54 (1H, dd, *J* 2.1 and *J*' 9.0 Hz), 7.51-7.45 (2H, m), 7.38-7.26 (8H, m), 7.12 (1H, d, *J* 9.0 Hz), 4.51 (2H, s); ESI (m/z, -c): 426.1 (16%) [M-H]⁻, 852.7 (100%) [2M-H]⁻, 875.2 [2(M-H)+Na]⁻, 1323.9 (10%) [3(M-H)+2Na]⁻.

### 4-{2-{4-[6'-Deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-2-oxoethyl}(phenyl)amino)-3-nitro-N-phenylbenzenesulfonamide 17c.

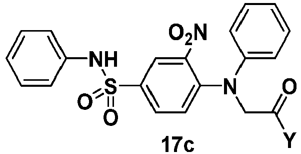

The synthesis of compound **17c** was carried out on 15.0 mmol scale of species **17b** (6.40 g) using anhydrous tetrahydrofuran (180 mL), compound **1a** (9.50 g, 16.5 mmol) and **10** (4.56 g, 16.5 mmol) according to "Method G". Compound **17c** (13.9 g, 93.9%) was obtained as foamy orange solid; *δ*_{H} (300 MHz, CDCl₃): 9.81 (1H, s), 8.53 (1H, d, *J* 2.1 Hz), 7.54 (1H, ddd, *J* 0.6 *J'* 2.4 and *J'* 9.2 Hz), 7.51-7.44 (2H, m), 7.38-7.29 (8H, m), 7.10 (1H, d, *J* 9.3 Hz), 4.51 (2H, s), 4.43-4.24 (4H, m), 4.19-3.99 (5H, m), 3.81-3.71 (2H, m), 3.71-3.41 (6H, m), 3.45 [6H, s (br)], 2.81-2.38 (6H, m), 1.51 (3H, s), 1.50 (3H, s), 1.40 (3H, s), 1.38 (3H, s), 1.35 (3H, s), 1.34 (3H, s).

### 4-{2-[4-(6'-Deoxy)(lactose-6'-yl)]-2-oxoethyl}(phenyl)amino)-3-nitro-N-phenyl-benzenesulfonamide hydrochloride 17d.

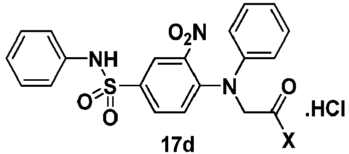

The synthesis of compound **17d** was carried out on 20.0 mmol scale of species **17c** (19.7 g) using acetone (240 mL) and hydrochloric acid (4.1 mL, 37% w/w, 50 mmol) dissolved in acetone (35 mL) according to "Method I". Compound **17d** (15.6 g, 91.2%) was obtained as yellow-orange solid, mp 180-195 °C (dec.); *λ*ₘₐₓ (ε) (H₂O): 420 nm (4200 M⁻¹cm⁻¹); *δ*_{H} (300 MHz, CDCl₃): 9.89 [1H, s (br)], 8.31-8.25 (1H, m), 7.72-7.61 (1H, m), 7.56-7.45 (2H, m), 7.45-7.21 (8H, m), 7.14 (1H, d, *J* 9.3 Hz), 5.23-4.52 (10H, m), 4.44-4.18 (2H, m), 4.14-3.96 (2H, m), 3.95-3.65 (3H, m), 3.64-3.54 (3H, m), 3.53-3.42 (2H, m), 3.41-2.81 (10H, m); ESI (m/z, +c): 820.5 (100%) [M+H]⁺, 842.4 (12%) [M+Na]⁺.

### 1,8-Dihydroxy-4-[4-(2-hydroxyethyl)phenylamino]-5-nitroanthracene-9,10-dione 18.

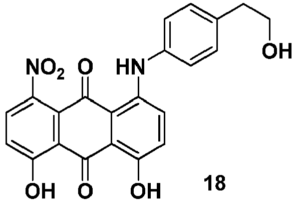

Commercial Disperse Blue 27 (Teratop Blue GLF, Huntsman) (100 g) was purified using dichloromethane (1.0 L) according to "Method A". Compound **18** (13.9 g, 13.9%) was obtained as dark blue solid, mp 94-95 °C; *δ*_{H} (200 MHz, CDCl₃): 11.70 (1H, s), 7.63-7.54 (2H, m), 7.32-7.10 (6H, m), 3.95-3.84 (2H, m), 2.97-2.83 (2H, m); ESI (m/z, -c): 419.3 (100%) [M-H]⁻, 861.3 (54%) [2(M-H)+Na]⁻.

### 4-{[4-(4,5-Dihydroxy-8-nitro-9,10-dioxo-9,10-dihydroanthracen-1-yl)amino]-phenethoxy}-4-oxobutanoic acid 18a.

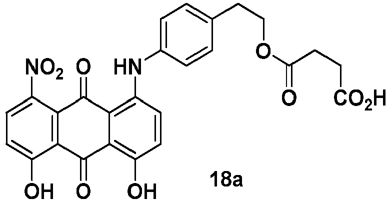

The synthesis of compound **18a** was carried out on 20 mmol scale of species **18** (8.40 g) using dichloromethane (100 mL), dihydrofuran-2,5-dione (2.40 g, 24 mmol), *N,N*-dimethylpyridin-4-amine (0.24 g, 2.0 mmol), triethylamine (2.63 g, 26 mmol) and acetic acid (6.0 g, 100.0 mmol) according to "Method E". Compound **18a** (9.1 g, 87.5%) was obtained as dark blue solid, mp 109-111.5 °C; *δ*_{H} (300 MHz, CDCl₃): 7.61 (1H, d, *J* 8.7 Hz), 7.56 (1H, d, *J* 9.6 Hz), 7.36-7.12 (6H, m), 4.34 (2H, t, *J* 6.6 Hz), 2.97 (2H, t, *J* 6.6 Hz), 2.72-2.58 (4H, m); ESI (m/z, -c): 519.5 (58%) [M-H]⁻, 1038.9 (100%) [2M-1]⁻.

### 4-(4,5-Dihydroxy-8-nitro-9,10-dioxo-9,10-dihydroanthracen-1-ylamino)phenethyl 4-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-4-oxobutanoate 18b.

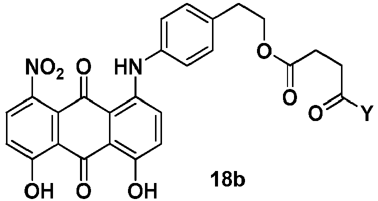

The synthesis of compound **18b** was carried out on 15.0 mmol scale of species **18a** (7.81 g) using anhydrous tetrahydrofuran (180 mL), compound **1a** (8.58 g, 16.5 mmol) and **10** (4.56 g, 16.5 mmol) according to "Method G". Compound **18b** (15.3 g, 94.4%) was obtained as foamy dark blue solid; *δ*_{H} (300 MHz, CDCl₃): 7.60 (1H, d, *J* 9.0 Hz), 7.56 (1H, d, *J* 9.9 Hz), 7.35-7.12 (6H, m), 4.45-4.22 (5H, m), 4.18-3.96 (6H, m), 3.95-3.79 (4H, m), 3.64-3.39 (4H, m), 3.44 (6H, s), 3.05-2.38 (12H, m), 1.49 (3H, s), 1.48 (3H, s), 1.37 (3H, s), 1.36 (3H, s), 1.32 (3H, s), 1.31 (3H, s).

### 4-(4,5-Dihydroxy-8-nitro-9,10-dioxo-9,10-dihydroanthracen-1-ylamino)phenethyl 4-[4-(6'-deoxy)(lactose-6'-yl)piperazin-1-yl]-4-oxobutanoate hydrochloride 18c.

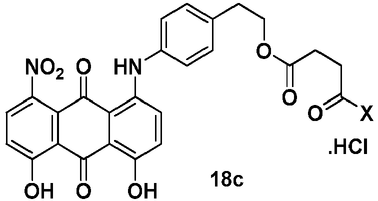

The synthesis of compound **18c** was carried out on 15.0 mmol scale of species **18b** (16.2 g) using acetone (160 mL) and hydrochloric acid (3.1 mL, 37% w/w, 37.5 mmol) dissolved in acetone (30 mL) according to "Method I". Compound **18c** (13.5 g, 94.8%) was obtained as dark blue solid, mp 160-185 °C (dec.); *λ*ₘₐₓ (ε) (H₂O): 600 nm (4800 M⁻¹cm⁻¹); *δ*_{H} (300 MHz, DMSO-d₆): 8.07 (1H, d, *J* 8.8 Hz), 7.64 (1H, d, *J* 9.9 Hz), 7.47 (1H, d, *J* 8.8 Hz), 7.42-7.23 (6H, m), 4.96-4.29 (5H, m), 4.28-4.19 (4H, m), 4.18-3.65 (16H, m), 3.64-3.41 (4H, m), 3.39-2.83 (5H, m), 2.72-2.37 (4H, m); ESI (m/z, +c): 913.6 (100%) [M+H]⁺.

### Ethyl 2-[9,10-dioxo-4-(p-tolylamino)-9,10-dihydroanthracen-1-yloxy]acetate 19.

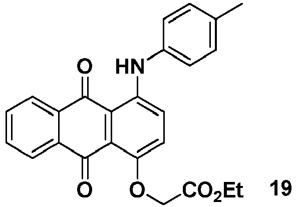

Disperse Blue 72 (0.99 g, 3.0 mmol, Orichem International Ltd.) was suspended in acetonitrile (10 mL) and potassium carbonate (0.91 g, 6.6 mmol) was added in one portion. The slurry was heated at 70 °C and ethyl 2-bromoacetate (0.90 g, 5.4 mmol) was added dropwise. The resulting mixture was brought to reflux for 5 h, after which it was cooled to 20 °C and diluted with water (100 mL). The fine suspension was aged for 0.5 h and it was filtered under suction. The press cake was washed with water and with petrol ether (bp: 40-60 °C) and dried in air. Compound **19** (1.19 g, 95.2%) was obtained as dark purple solid, mp 142-145 °C; *δ*_{H} (200 MHz, CDCl₃): 11.50 (1H, s), 8.33-8.19 (2H, m), 7.83-7.68 (2H, m), 7.48-7.39 (1H, m), 7.34-7.12 (5H, m), 4.74 (2H, s), 4.28 (2H, q, *J* 7.2 Hz), 2.38 (3H, s), 1.31 (3H, t, *J* 7.2 Hz); ESI (m/z, +c): 416.2 (3%) [M+H]⁺, 438.3 (4%) [M+Na]⁺, 853.4 (100%) [2M+Na]⁺.

### 2-[9,10-Dioxo-4-(p-tolylamino)-9,10-dihydroanthracen-1-oxyl]acetic acid 19a.

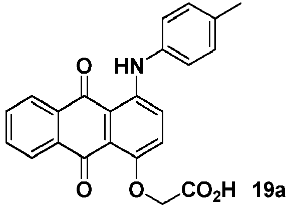

The synthesis of compound **19a** was carried out on 1.88 mmol scale of species **19** (0.78 g) using tetrahydrofuran (20 mL), an aq. 1 M solution of potassium hydroxide (0.21 g, 3.76 mmol) and 1 M hydrochloric acid according to "Method D". Compound **19a** (0.69 g, 94.5%) was obtained as purple solid; *δ*_{H} (200 MHz, CDCl₃): 11.36 (1H, s), 8.30-8.05 (2H, m), 7.95-7.80 (2H, m), 7.59-7.39 (2H, m), 7.33-7.13 (4H, m), 4.79 (2H, s), 2.33 (3H, s).

### (S)-Diethyl 2-{2-[9,10-dioxo-4-(p-tolylamino)-9,10-dihydroanthracen-1-oxyl]-acetamido}pentanedioate 19b.

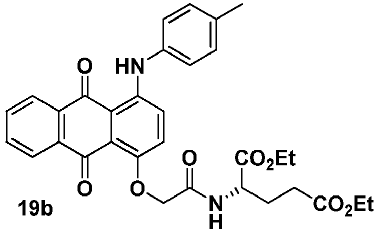

The synthesis of compound **19b** was carried out on 1.73 mmol scale of species **19a** (0.67 g) using anhydrous tetrahydrofuran (20 mL), (*S*)-diethyl 2-aminopentanedioate hydrochloride (0.46 g, 1.90 mmol), 4-methylmorpholine (0.19 g, 1.90 mmol) and compound **10** (0.53 g, 1.90 mmol) according to "Method H". Compound **19b** (0.91 g, 91.9%) was obtained as red solid, mp 73-75 °C; *δ*_{H} (200 MHz, CDCl₃): 11.48 (1H, s), 9.27 [1H, d (br), *J* 7.8 Hz], 8.33-8.20 (2H, m), 7.83-7.68 (2H, m), 7.56-7.42 (1H, m), 7.30-7.08 (5H, m), 4.78-4.64 (1H, m), 4.66 (2H, s), 4.25 (2H, q, *J* 7.2 Hz), 4.16 (2H, q, *J* 7.2 Hz), 2.73-2.58 (2H, m), 2.45-2.30 (2H, m), 2.39 (3H, s), 1.31 (3H, t, *J* 7.2 Hz), 1.26 (3H, t, *J* 7.2 Hz); ESI (m/z, +c): 573.2 (7%) [M+H]⁺, 595.4 (9%) [M+Na]⁺, 1167.4 [2M+Na]⁺.

### (S)-2-{2-[9,10-dioxo-4-(p-tolylamino)-9,10-dihydroanthracen-1-oxyl]acetamido}pentanedioic acid 19c.

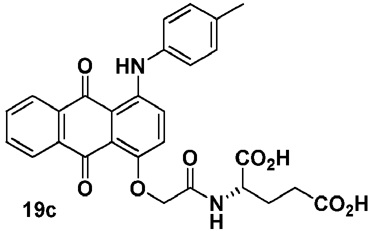

The synthesis of compound **19c** was carried out on 2.8 mmol scale of species **19b** (1.64 g) using tetrahydrofuran (30 mL), an aq. 1 M solution of potassium hydroxide (0.63 g, 11.2 mmol) and 1 M hydrochloric acid according to "Method D". Compound **19c** (1.4 g, 96.6%) was obtained as dark purple solid, mp 153-156 °C; *δ*_{H} (200 MHz, DMSO-d₆): 11.40 (1H, s), 8.86 [1H, d (br), *J* 7.8 Hz], 8.34-8.06 (2H, m), 8.03-7.79 (2H, m), 7.64-7.40 (1H, m), 7.35-7.09 (5H, m), 4.18-4.58 [2H, (AB)], 4.48-4.28 (1H, m), 2.57-2.40 (2H, m), 2.34 (3H, s), 2.22-1.96 (2H, m); ESI (m/z, -c): 515.1 (6%) [M-H]⁻, 1030.7 (100%) [2M-H]⁻.

### N-{(S)-1,5-Bis-{4-[6'-deoxy-1'-(2,3:5,6-di-O-isopropylidene-1,1-dimethoxyaldehydo-D-glucos-4-yl)-3',4'-O-(isopropylidene)-β-D-galactopyranos-6'-yl]piperazin-1-yl}-1,5-dioxopentan-2-yl}-2-[9,10-dioxo-4-(p-tolylamino)-9,10-dihydroanthracen-1-oxyl]acetamide 19d.

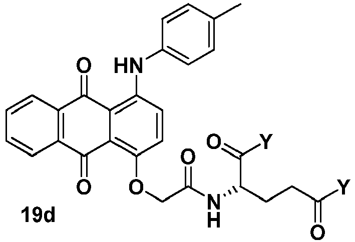

The synthesis of compound **19d** was carried out on 1.0 mmol scale of species **19c** (0.52 g) using anhydrous tetrahydrofuran (15 mL), compound **1a** (1.27 g, 2.2 mmol) and **10** (0.61 g, 2.2 mmol) according to "Method G". Compound **19d** (1.41 g, 86.5%) was obtained as foamy purple solid; *δ*_{H} (200 MHz, CDCl₃): 11.47 (1H, s), 9.02 [1H, d (br), *J* 7.8 Hz], 8.43-8.19 (2H, m), 7.91-7.67 (2H, m), 7.55-7.41 (1H, m), 7.38-7.03 (5H, m), 5.24-5.05 (1H, m), 4.68-4.50 [2H, (AB)], 4.46-4.21 (4H, m), 4.20-3.95 (14H, m), 3.91-3.68 (8H, m), 3.66-3.33 (20H, m), 2.82-2.23 (16H, m), 2.38 (3H, s), 1.78 [1H, s (br)], 1.49 [12H, s (br)], 1.39-1.30 (24H, m).

### N-{(S)-1,5-Bis-[4-(6'-deoxy)(lactose-6'-yl)piperazin-1-yl]-1,5-dioxopentan-2-yl}-2-[9,10-dioxo-4-(p-tolylamino)-9,10-dihydroanthracen-1-oxyl]acetamide ditrifluoroacetate 19e.

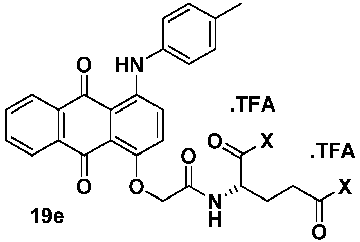

The synthesis of compound **19e** was carried out on 0.86 mmol scale of species **19d** (1.41 g) using 90% aq. (v/v) 2,2,2-trifluoroacetic acid (30 mL), toluene and acetone, according to "Method J". Compound **19e** (1.19 g, 90.8%) was obtained as dark purple solid, mp 150-175 °C (dec.); *λ*ₘₐₓ (ε) (H₂O): 555 nm (7300 M⁻¹cm⁻¹); *δ*_{H} (200 MHz, DMSO-d₆): 11.15 [1H, s (br)], 8.38 [1H, m (br)], 8.28-8.05 (2H, m), 7.98-7.81 (2H, m), 7.63-7.48 (2H, m), 7.39-7.18 (6H, m), 5.47-4.61 (9H, m), 4.53-4.22 (8H, m), 4.17-3.49 (33H, m), 3.45-3.95 (16H, m), 2.64-2.23 (4H, m); ESI (m/z, +c): 652.2 (80%) [M+2H]²⁺, 1301.7 (100%) [M+H]⁺.

### TINCTORIAL AND FASTNESS TESTS

### Leather dyeing

Leather samples of the Box Calf type were dyed following a retanning/dyeing protocol, including: a retanning procedure to refine the leather fullness and softness, a fatliquoring procedure to improve the mechanical properties of the leather and a dyeing procedure to give the desired color to the leather. All these procedures were summarized as shown below:

| **Phase** | **Weight** | **Chemical** | **Temp.** | **Rotation (min)** | |
|---|---|---|---|---|---|
| **Retanning** | 200% | Water | 35 °C | | |
| | 0,5% | Tanning auxiliary for leather bleaching | | 20 | |
| | | | | | Drain |
| | 200% | Water | 35 °C | 10 | |
| | | | | | Drain |
| | 100% | Water | 35 °C | 10 | |
| | 1.0% | Sodium bicarbonate | | | |
| | 0.5% | Sodium formate | | 90 | |
| | | | | | Drain |
| | 200% | Water | 35 °C | 10 | |
| | | | | | Drain |
| | 100% | Water | 25 °C | | |
| | 3% | Synthetic tannins for light fastness | | 60 | |
| | 200% | Water | 45 °C | 10 | |
| | | | | | Drain |
| **Fatliquoring** | 150% | Water | 50 °C | | |
| | 5% | Sulphatated synthetic fatliquor | | 60 | |
| | 1% | Formic acid | | 20 | |
| | | | | | Drain |
| | 200% | Water | 25 °C | 10 | |
| | | | | | Drain |
| **Dyeing** | 70% | Water | 25 °C | | |
| | 3% | Dye | | 60 | |
| | 100% | Water | 50 °C | | |
| | 2% | Formic acid | | 30 | |
| | | | | | Drain |
| | 200% | Water | 25 °C | 10 | |
| | | | | | Drain |

According to the above procedure, dyed leather samples were obtained using compounds: **2d, 3d, 4b, 5b, 6b, 7b, 8b, 9b, 11d, 12d, 14b, 17d, 18c** and **19e.** The results for colors and fastness tests were reported in **Table 1:**

**Table 1**

| **Dye** | **Hue** | **Light** | **PVC** | **Wet Rubbing** |
|---|---|---|---|---|
| **2d** | Deep Orange | 2-3 | 3 | 2-3 |
| **3d** | Orange | 2-3 | 4 | 3-4 |
| **4b** | Red-Light Brown | 3 | 3-4 | 2-3 |
| **5b** | Dark Red | 3-4 | 1-2 | 1-2 |
| **6b** | Pale Yellow | 7-8 | 4-5 | 4-5 |
| **7b** | Pale Pink | 5 | 3-4 | 4 |
| **8b** | Light Pink | 3-4 | 2-3 | 3-4 |
| **9b** | Pale Pink | 2-3 | 4-5 | 3-4 |
| **11d** | Deep Violet | 2-3 | 3 | 3-4 |
| **12d** | Orange | 4 | 4-5 | 4 |
| **14b** | Light Orange | 2 | 3 | 3 |
| **17d** | Yellow-Green | 7-8 | 5 | 5 |
| **18c** | Blue | 7 | 4 | 4 |
| **19e** | Light Violet | 5 | 4 | 4 |

### Hair Dyeing

Hair samples of white yak hair type were dyed using a preformulated paste for hair dyeing tests and a dye (1% w/w) of the formula (IIIe). Dyeing tests were carried out at 25 °C keeping hair samples in contact with the dyeing paste for 0.5 h and containing formic acid to pH 2, acetic acid to pH 3-4 and a phosphate buffer to pH 6-7.

According to the above method, dyed hair samples were obtained using compounds: **4b, 13c** and **18c** and the results for colors and washing tests were reported in **Table 2:**

**Table 2**

| **Dye** | **Hue** | **pH** | **3 Washes** | **6 Washes** | **9 Washes** | **12 Washes** |
|---|---|---|---|---|---|---|
| **4b** | Brick Red | 2 | 3 | 3 | 2 | 1-2 |
| **4b** | | 3-4 | 3-4 | 3 | 2-3 | 2 |
| **4b** | | 6-7 | 3 | 3 | 2 | 1 |
| **13c** | Bright Yellow | 2 | 4-5 | 4 | 3-4 | 3 |
| **13c** | | 3-4 | 4 | 4 | 3-4 | 3 |
| **13c** | | 6-7 | 4 | 4 | 3-4 | 3 |
| **18c** | Light Violet | 2 | 4 | 4 | 3-4 | 2-3 |
| **18c** | | 3-4 | 4 | 3-4 | 3 | 2 |
| **18c** | | 6-7 | 3-4 | 3-4 | 3 | 2 |

### WOOD DYEING

Wood samples of fir wood type were soaked in tap water containing a dye (1.5% w/w) of the formula (IIIe). Dyeing was carried out for 16 h at 20-25 °C, after which the bath was decanted and the sample were rinsed with cold water and dried. According to the above method, dyed wood samples were obtained using compounds: **4b, 5b, 12d** and **18c** and the results for colors and light fastness were reported in **Table 3:**

**Table 3**

| **Dye** | **Hue** | **Light** |
|---|---|---|
| **4b** | Red-Light Brown | 5 |
| **5b** | Red | 5 |
| **12d** | Bright Orange | 5-6 |
| **18c** | Dark Turquoise | 7-8 |

### POLYESTER FABRIC DYEING

Fabric samples of polyester (10 g) were dyed at 98 °C for 0.75 h, using a 13:1 (v/w) water bath containing 0.1% (w/w) of dye and acetic acid (aq. 10% w/w) to reach pH 4. The temperature of the bath was brought to 20-25 °C, the dye bath was decanted and fabric samples were rinsed with cold water and dried. A portion of the dyed fabric was subjected to a washing protocol, using a mild non ionic detergent at 60 °C for 0.5 h, rinsed with cold water and dried.

According to the present invention, dyed polyester fabric samples were obtained using compounds **2d, 4b, 6b, 8b, 11d, 12d, 15a, 16c, 17d** and **18c** and the results for colors and fastness tests were reported in **Table 4:**

**Table 4**

| **Dye** | **Hue** | **Light** | **Washing*** | **Rubbing** | | **Sublimation** |
|---|---|---|---|---|---|---|
| | | | | **Dry** | **Wet** | |
| **2d** | orange | 3 | 4 | 4-5 | 4 | 5 |
| **4b** | red | 3-4 | 3-4 | 4 | 3-4 | 4-5 |
| **6b** | yellow | 5-6 | 5 | 5 | 5 | 5 |
| **8b** | red | 4-5 | 3-4 | 4 | 3-4 | 4-5 |
| **11d** | violet | 3 | 4 | 4 | 3-4 | 5 |
| **12d** | orange | 5 | 4-5 | 4-5 | 4-5 | 5 |
| **15a** | orange | 3-4 | 4-5 | 4-5 | 4-5 | 5 |
| **16c** | yellow | 4 | 4-5 | 4-5 | 4-5 | 5 |
| **17d** | yellow | 6-7 | 5 | 5 | 5 | 5 |
| **18c** | blue | 6-7 | 4-5 | 4 | 4 | 4-5 |

| | | | | | | |
|---|---|---|---|---|---|---|
| ***: at 60 °C with a non ionic detergent** | | | | | | |

Fabric samples of polyester (5 g) were dyed at 98 °C for 0.75 h, using a 20:1 (v/w) water bath containing 1% (w/w) of dye and potassium carbonate to reach pH 4. The temperature of the bath was brought to 20-25 °C, the dye bath was decanted and fabric samples were rinsed with cold water and dried. A portion of the dyed fabric was subjected to a washing protocol, using a mild non ionic detergent at 60 °C for 0.5 h, rinsed with cold water and dried.

According to the present invention, dyed polyester fabric samples were obtained using compounds **12d** and **18c** and the results for colors and fastness tests were reported in **Table 5:**

**Table 5**

| **Dye** | **Hue** | **Light** | **Washing*** | **Rubbing** | | **Sublimation** |
|---|---|---|---|---|---|---|
| | | | | **Dry** | **Wet** | |
| **12d** | orange | 7 | 5 | 5 | 5 | 5 |
| **18c** | blue | 6-7 | 5 | | 4-5 | 5 |

| | | | | | | |
|---|---|---|---|---|---|---|
| ***: at 60 °C with a non ionic detergent** | | | | | | |

## Claims

1. A compound of formula (I) 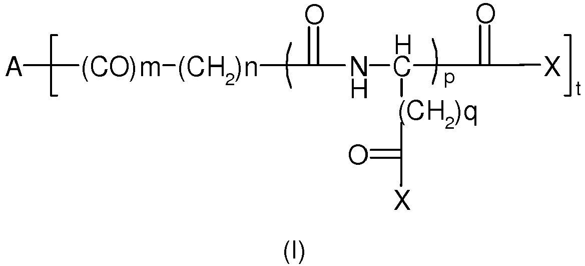 wherein
X is 6'-deoxy-6'-(piperazinyl)lactose
**X =** 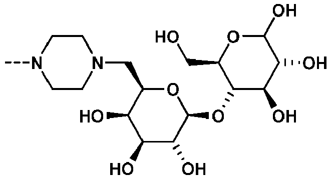
A is a chromophore moiety;
p=0, 1 and when p=1 then q=1, 2;
n=0, 1, 2, 3;
m=0, 1;
t=1, 2 proviso that is t=1 when p=1;
and wherein all possible optical isomers are included, such as enantiomers and/or diastereoisomers, mixtures thereof, both as racemes or in various ratios, and inorganic or organic salts.

2. Compound according to claim 1 wherein the chromophore moiety is selected in the group consisting of diaryl-azoic chromophores, anthraquinone chromophores and aniline chromophores.

3. Compound according to anyone of the preceding claims wherein the chromophore moiety is bonded to the rest of the molecule through a terminal function selected in the group consisting of a primary or secondary amine, an hydroxy group and a carboxylic function thus giving place to an amino linkage, an ether linkage, an amide linkage or and ester linkage.

4. Compound according to anyone of claims 1-3 wherein p=0 therefore resulting in a compound of formula (III) 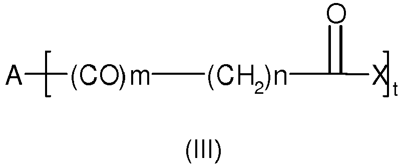 wherein n=0, 1, 2, 3; m=0, 1; t=1, 2.

5. Compound according to anyone of claims 1-3 wherein p=1, then t=1, therefore resulting in a compound of formula (IV): 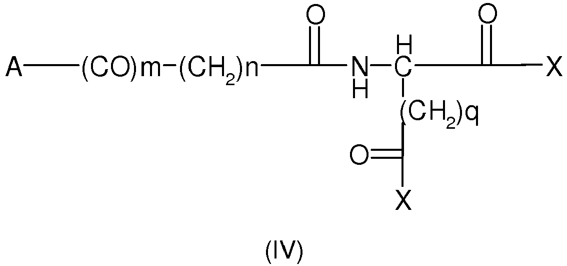 wherein q=1, 2; n=0, 1, 2, 3; m=0, 1.

6. A compound of formula (II) 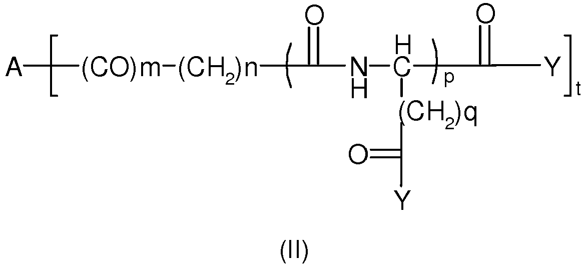 wherein
Y= 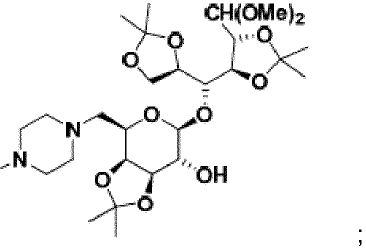 and A, p, q, n, m and t are as defined in any one of claims 1-5.

7. Use of a compound of formula (II) according to claim 6 as intermediate for the preparation of a compound of formula (I) according to any one of claims 1-5.

8. A process for preparing a compound of formula (I) as above described, said process comprising the following step:
(b) deprotecting of the acetonide hydroxy functions of the 6'-deoxy-6'-(piperazinyl)lactose moiety, of a compound of formula (II) according to claim 6.

9. Use of a compound of formula (I) according to any one of claims 1-5 as dyeing agent.

10. Use according to claim 9 for dyeing textiles, leather, wood and hair.

11. A dyeing composition comprising a compound of formula (I) according to anyone of claims 1-5.

12. A method for dyeing wood, leather or hair wherein as dyeing agent is used a compound of formula (I) wherein 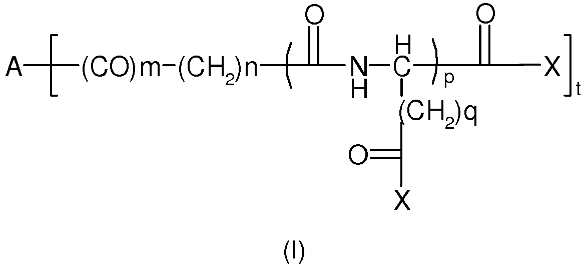 wherein
X is 6'-deoxy-6'-(piperazinyl)lactose or 6'-deoxy-6'-amino-lactose
A is a chromophore moiety;
p=0, 1 and when p=1 then q=1, 2;
n=0, 1, 2, 3;
m=0, 1;
t=1, 2 proviso that is t=1 when p=1;
and wherein all possible optical isomers are included, such as enantiomers and/or diastereoisomers, mixtures thereof, both as racemes or in various ratios, and inorganic or organic salts.

13. A method comprising dyeing polyester at a temperature comprised between 95 and 135°C, at atmospheric pressure in pH conditions ranging from 4 to 10 and in absence of dyeing auxiliaries, said method wherein is used a compound of formula (I) according to claim 12.

14. A hair dyeing composition comprising a compound of formula (I) according to claim 12.

## Patentansprüche

1. Zusammensetzung der Formel (I) 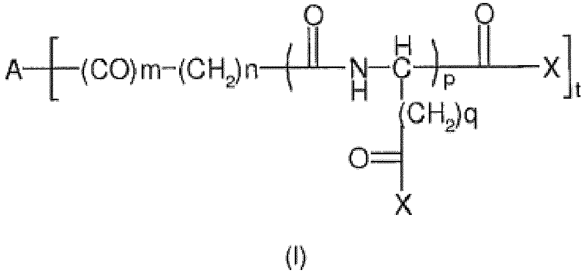 wobei
X eine 6'-deoxy-6'-(Piperazinyl)Lactose ist
**X =** 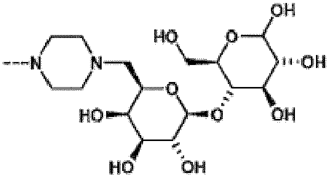
A ein chromophorer Teil ist;
p=0, 1 und wenn p=1 dann q=1, 2;
n=0, 1, 2, 3;
m=0, 1;
t=1, 2 vorbehaltlich, dass t=1 ist, wenn p=1 ist;
und wobei alle möglichen optischen Isomere beinhaltet sind, wie Enantiomere und/oder Diastereoisomere, Mischungen davon, beide "*Trauben*"*-*artig oder in verschiedenen Verhältnissen, sowie anorganische oder organische Salze.

2. Zusammensetzung nach Anspruch 1, wobei der chromophore Teil aus der Gruppe bestehend aus Diaryl-Azo-Chromophor, Anthrachinon-Chromophor und Anilin-Chromophor ausgewählt ist.

3. Zusammensetzung nach einem der vorangegangenen Ansprüche, wobei der chromophore Teil an den Rest des Moleküls durch eine terminale Funktion ausgewählt aus der Gruppe bestehend aus einem primären oder sekundären Amin, einer HydroxyGruppe und einer Karboxyl-Funktion gebunden ist und so eine Stelle frei ist für eine Amin-Verbindung, eine Äther-Verbindung, eine Amid-Verbindung oder einer Ester-Verbindung.

4. Zusammensetzung nach einem der Ansprüche 1 bis 3, wobei p=0 ist und daher eine Zusammensetzung der Formel (III) resultiert 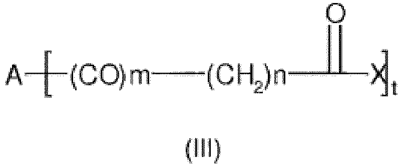 wobei n=0, 1, 2, 3; m=0, 1; t=1, 2 ist.

5. Zusammensetzung nach einem der Ansprüche 1 bis 3, wobei p=1 ist, dann t=1 ist, womit eine Mischung der Formel (IV) resultiert: 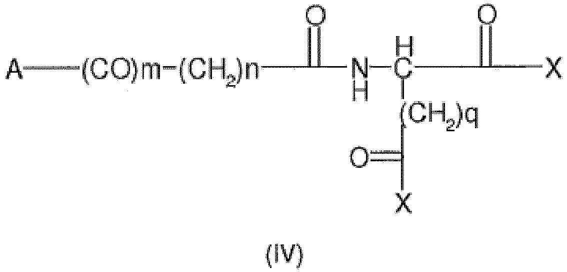 wobei q=1, 2; n=0, 1, 2, 3; m=0, 1 ist.

6. Eine Zusammensetzung der Formel (II) 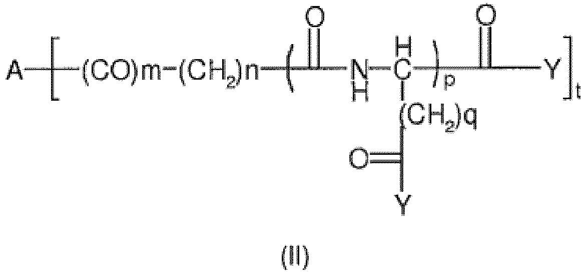 wobei 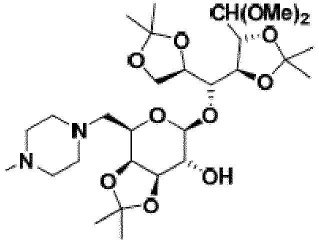 Y=
und A, p, q, n, m und t definiert sind gemäß einem der Ansprüche 1 bis 5.

7. Verwendung der Zusammensetzung der Formel (II) nach Anspruch 6 als Zwischenformel für die Zubereitung einer Zusammensetzung der Formel (I) nach einem der Ansprüche 1 bis 5.

8. Verfahren zum Herstellen einer Zusammensetzung der Formel (I) wie oben beschrieben, wobei das Verfahren die folgenden Schritte aufweist:
(b) Deprotektierung der Acetonid-Hydroxy-Funktionen des 6'-deoxy-6'-(Piperazinyl) Lactoseteils einer Zusammensetzung der Formel (II) nach Anspruch 6.

9. Verwendung einer Zusammensetzung der Formel (I) nach einem der Ansprüche 1 bis 5 als Färbemittel.

10. Verwendung nach Anspruch 9 zum Färben von Textilien, Leder, Holz und Haaren.

11. Eine Färbezusammensetzung aufweisend eine Zusammensetzung der Formel (I) nach einem der Ansprüche 1 bis 5.

12. Ein Verfahren zum Färben von Holz, Leder oder Haaren, wobei als Färbemittel eine Zusammensetzung der Formel (I) verwendet wird, wobei 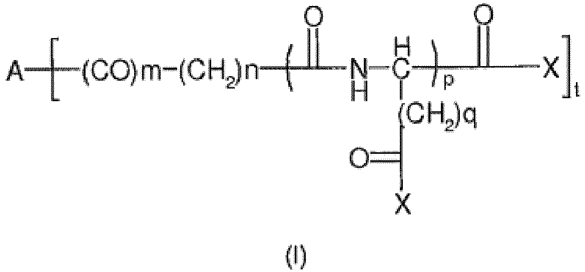 wobei
X= 6'-deoxy-6'-(Piperazinyl) Lactose oder 6'-deoxy-6'-amino-Lactose ist;
A ein chromophorer Teil ist;
p=0, 1 und wenn p=1 dann q=1, 2;
n=0, 1, 2, 3;
m=0, 1;
t=1, 2 vorausgesetzt, dass t=1 ist, wenn p=1 ist;
und wobei alle möglichen optischen Isomere eingeschlossen sind, wie Enantiomere und/oder Diastereoisomere, Mischungen davon, beide "*Trauben*"*-*artig oder in verschiedenen Verhältnissen, sowie anorganische oder organische Salze.

13. Verfahren aufweisend einen Färbepolyester mit einer Temperatur aufweisend zwischen 95 und 135°C, bei Umgebungsdruck in einem pH-Zustand zwischen 4 und 10 und in Abwesenheit von Färbehilfsmitteln, wobei das Verfahren eine Zusammensetzung der Formel (I) nach Anspruch 12 verwendet.

14. Haarfärbezusammensetzung aufweisend eine Zusammensetzung der Formel (I) nach Anspruch 12.

## Revendications

1. Composé de formule (I) 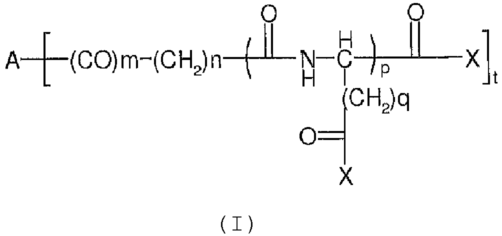 dans laquelle
X représente un 6'-désoxy-6'(pipérazinyl)lactose
**X =** 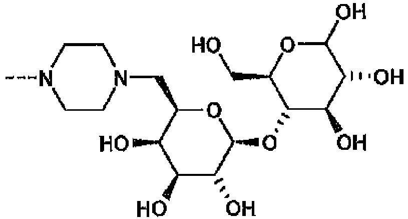
A représente une fraction chromophore ;
p = 0, 1 et lorsque p = 1 alors q = 1, 2 ;
n = 0, 1, 2, 3 ;
m = 0, 1 ;
t = 1, 2 à condition que t = 1 lorsque p = 1 ;
et dans laquelle sont inclus tous les isomères optiques possibles, tels que des énantiomères et/ou des diastéréoisomères, leurs mélanges, en tant que racèmes ou selon divers rapports, et des sels organiques ou inorganiques.

2. Composé selon la revendication 1 dans lequel la fraction chromophore est choisie dans le groupe constitué de chromophores diaryl-azoïques, de chromophores anthraquinones et chromophores anilines.

3. Composé selon l'une quelconque des revendications précédentes dans lequel la fraction chromophore est liée au reste de la molécule par une fonction terminale choisie dans le groupe constitué d'une amine primaire ou secondaire, d'un groupe hydroxy et d'une fonction carboxylique donnant ainsi lieu à une liaison amine, une liaison éther, une liaison amide ou une liaison ester.

4. Composé selon l'une quelconque des revendications 1 à 3 dans lequel p = 0, ayant pour résultat un composé de formule (III) 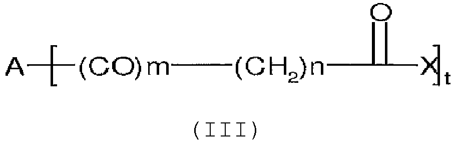 dans laquelle n = 0, 1, 2, 3 ; m = 0, 1 ; t = 1, 2.

5. Composé selon l'une quelconque des revendications 1 à 3 dans lequel p = 1, donc t = 1, ayant pour résultat un composé de formule (IV) : 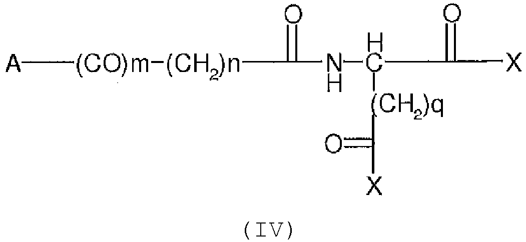 dans laquelle q = 1, 2 ; n = 0, 1, 2, 3 ; m = 0, 1.

6. Composé de formule (II) 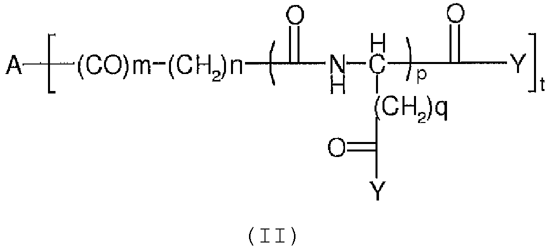 dans laquelle
Y= 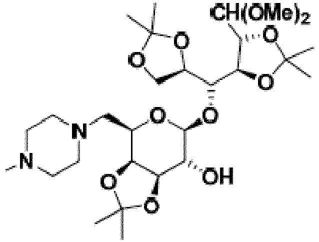 et A, p, q, n, m et t sont tels que définis dans l'une quelconque des revendications 1 à 5.

7. Utilisation d'un composé de formule (II) selon la revendication 6 comme intermédiaire pour la préparation d'un composé de formule (I) selon l'une quelconque des revendications 1 à 5.

8. Procédé de préparation d'un composé de formule (I) tel que décrit ci-dessus, ledit procédé comprenant l'étape suivante :
(b) déprotection des fonctions hydroxy acétonide de la fraction 6'-désoxy-6'(pipérazinyl)lactose d'un composé de formule (II) selon la revendication 6.

9. Utilisation d'un composé de formule (I) selon l'une quelconque des revendications 1 à 5 comme agent de coloration.

10. Utilisation selon la revendication 9 pour teindre des textiles, du cuir, du bois et des cheveux.

11. Composition de coloration comprenant un composé de formule (I) selon l'une quelconque des revendications 1 à 5.

12. Procédé de teinture de bois, de cuir ou de cheveux dans lequel on utilise comme agent de coloration un composé de formule (I) dans lequel 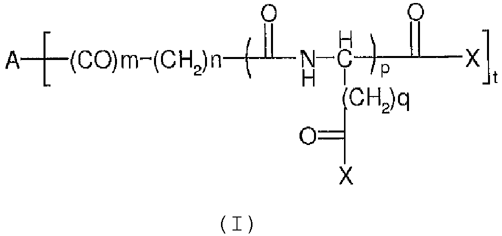 dans laquelle
X représente un 6'-désoxy-6'(pipérazinyl)lactose ou un 6'-désoxy-6'-amino-lactose
A représente une fraction chromophore ;
p = 0, 1 et lorsque p = 1 alors q = 1, 2 ;
n = 0, 1, 2, 3 ;
m = 0, 1 ;
t = 1, 2 à condition que t = 1 lorsque p = 1 ;
et dans laquelle sont inclus tous les isomères optiques possibles, tels que des énantiomères et/ou des diastéréoisomères, leurs mélanges, en tant que racèmes ou selon divers rapports, et des sels organiques ou inorganiques.

13. Procédé comprenant la teinture d'un polyester à une température comprise entre 95 et 135 °C, à pression atmosphérique dans des conditions de pH allant de 4 à 10 et en l'absence d'auxiliaires de coloration, ledit procédé dans lequel on utilise un composé de formule (I) selon la revendication 12.

14. Composition de coloration capillaire comprenant un composé de formule (I) selon la revendication 12.
